# FASCICULE DE BREVET EUROPEEN

(11) **EP 1 456 229 B1**
(45) Date de publication et mention de la délivrance du brevet: **02.09.2009**
(21) Numéro de dépôt: 02805374.2
(22) Date de dépôt: 12.12.2002
(51) Int. Cl.: C07K 7/56, A61K 38/12, A61P 31/10

(54) **PROCEDE DE PREPARATION DE DERIVES D ECHINOCANDINE**
VERFAHREN ZUR HERSTELLUNG VON ECHINOCANDINDERIVATEN
METHOD FOR PREPARING ECHINOCANDIN DERIVATIVES

(30) Priorité: 14.12.2001 FR 0116230
(43) Date de publication de la demande: 15.09.2004
(73) Titulaire: NOVEXEL, 93230 Romainville (FR)
(72) Inventeur: BOFFELLI, Philippe, F-93110 Rosny sous Bois (FR); BROUILLARD, Agnès, F-93250 Villemomble (FR); COLLADANT, Colette, F-93110 Rosny sous Bois (FR); DROUX, Serge, F-77230 Dammartin en Goële (FR); ELTER, Michel, F-95410 Groslay (FR); FERROUD, Didier, F-93700 Drancy (FR); LEMAITRE, Guy, F-77280 Othis (FR); PALADINO, Joseph, F-01600 Parcieux (FR)
(74) Mandataire: Hirsch & Associés
(86) Numéro de dépôt international: PCT/FR2002/004308
(87) Numéro de publication internationale: WO 2003/054001

(56) Documents cités:
- WO-A-00/75177
- WO-A-01/07468
- WO-A-99/29716

## Description

La présente invention a pour objet un procédé de préparation de dérivés d'échinocandine, les intermédiaires et les produits obtenus et leur application comme médicament antifongique.

De nombreux composés ayant une activité antifongique sont connus dans l'art antérieur. On peut citer notamment les dérivés 4- amino de l'échinocandine tels que définis dans la demande internationale WO99/29716 et notamment la 1-[4-[(2-aminoéthyl) amino] - N2- [[4'-(octyloxy) [1,1' - biphényl] - 4 - yl] carbonyl] - L - ornithine] -4 -[4 - (4 - hydroxyphényl) - L thréonine] - 5 - L - sérine échinocandine B sous la forme 4S ou 4R ou sous la forme d'un mélange des stéréoisomères et des analogues acyles, ainsi que leurs sels d'addition avec les acides.

L'objectif de la présente invention est de fournir un nouveau procédé de préparation de dérivés de l'échinocandine de ce type.

L'échinocandine peut se présenter sous la forme de deux épimères appelés isomère A et isomère B correspondant aux configurations R et S du substituant en position 4.
Un des objets de la présente invention est d'obtenir au cours du procédé, l'un des deux isomères de manière majoritaire, c'est-à-dire au moins supérieur à 50% (isomère appelé forme A, correspondant au composé de formule (I) pharmacologiquement actif), ce qui n'est pas le cas du procédé selon WO99/29716 au cours duquel l'isomère actif A obtenu au cours du procédé avant purification sur colonne chromatographique est minoritaire.
Par ailleurs un autre objectif de la présente invention est d'éviter les purifications des produits intermédiaires par chromatographie et d'obtenir des produits cristallisés ce qui permet d'améliorer de manière significative les rendements. L'invention a donc pour objet un procédé de préparation de composés de formule (I) 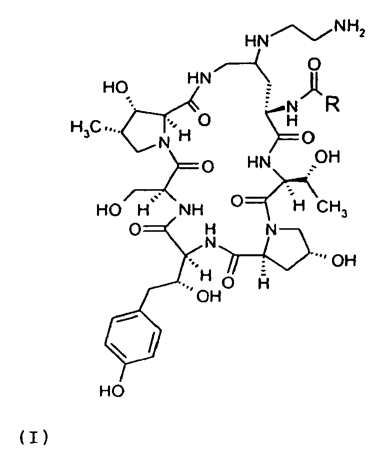 dans laquelle R représente une chaîne linéaire ou ramifiée ou cyclique renfermant jusqu'à 30 atomes de carbone, renfermant éventuellement un ou plusieurs hétéroatomes et un ou plusieurs hétérocycles
comprenant les étapes suivantes
a) On soumet un composé de formule (II) 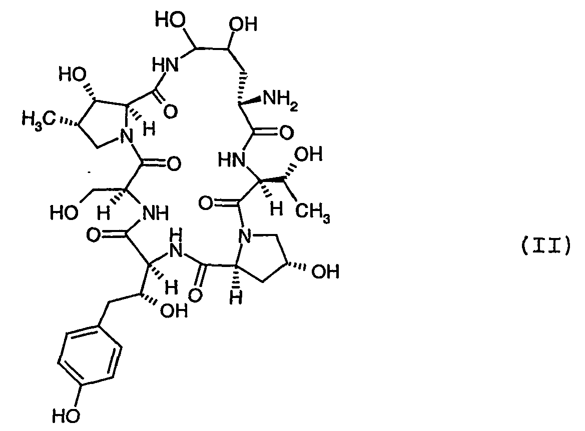 à l'action d'un acide de formule R-CO₂H, R étant tel que défini plus haut, le dit acide étant le cas échéant sous une forme activée isolée ou non isolée, afin d'obtenir le composé de formule (III) 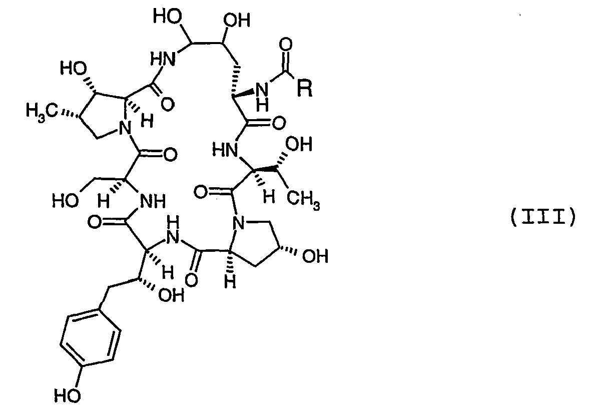
b) le cas échéant, on soumet le composé de formule (III) à une réaction d'alkylation de l'alcool en position 5 par action d'un alcool de formule Alk-OH en présence de PPTS, Alk étant un radical Alkyle renfermant de 1 à 4 atomes de carbone, afin d'obtenir le composé de formule (III') 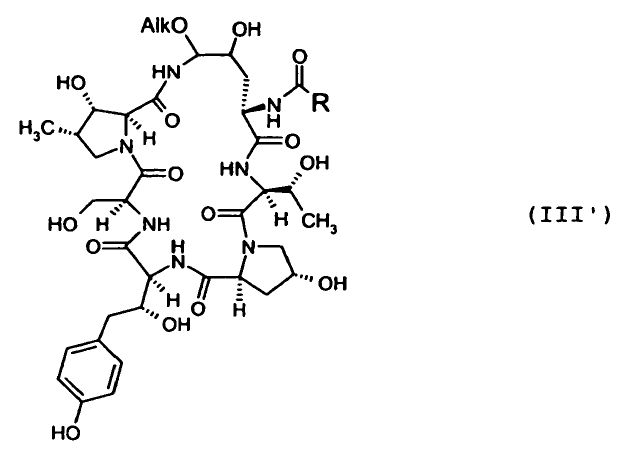
c) on soumet le composé de formule (III) ou (III') à une réaction de déshydratation, afin d'obtenir un composé de formule (IV) 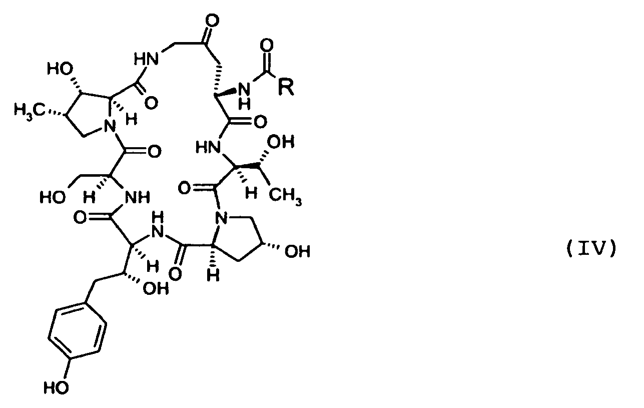
d) on soumet le composé de formule (IV) à une réaction d'amination réductrice par action d'éthylène diamine en présence d'un agent réducteur tel que le NaBH₃CN en présence d'un acide de Lewis, ou le NaBH(OCOR')₃, OCOR' représentant la Boc-L-Pro, la Bzl-L-Pro ou tout autre aminoacide optiquement actif ainsi que tout autre acide carboxylique chiral ou non, afin d'obtenir le composé de formule (I) tel que défini plus haut, comportant majoritairement l'un des isomères actifs,
   le dit composé de formule (I) étant par la suite soumis le cas échéant et dans un ordre approprié à l'une ou plusieurs des opérations suivantes
   - purification par chromatographie
   - purification par Cristallisation
   - action d'une base
   - salification.

De préférence l'invention a pour objet un procédé tel que défini précédemment dans lequel les composés de formules (I), (III), (III') ou (IV) renferment un radical R représentant les groupements suivants :

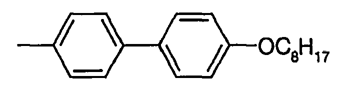

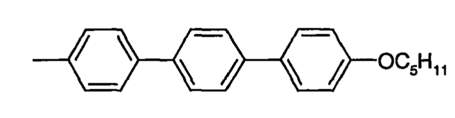

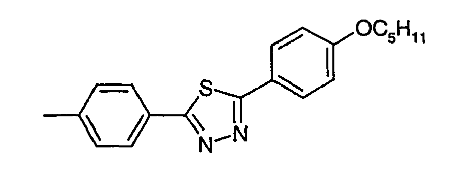

De préférence, l'invention a pour objet un procédé tel que défini précédemment dans lequel les composés de formules (I), (III), (III') ou (IV) renferment un radical R représentant le groupement suivant :

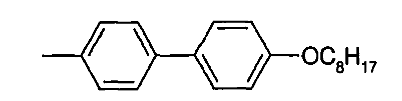

Le composé de formule (II) est préparé selon la méthode décrite dans la demande internationale WO99/29716 page 18 (préparation 2, "nucleus de déoxymulundocandine").

L'activation de l'acide de formule R-CO₂H s'effectue selon les méthodes classiques connues de l'homme du métier (Journet M. et al, J. Org. Chem. (1999), 64, 2411-2417; Pöchlauer P. et al. Tetrahedron 54(1998) 3489-3494; Kunushima et al. Tetrahedron (1999) 55 13159-13170). En particulier on utilise le pentaflurophénol afin d'obtenir l'ester activé de pentafluorophénol qui est isolé avant d'être utilisé dans la réaction d'acylation de l'amine. Une autre méthode est l'utilisation de N-hydroxysuccinimide (éventuellement N-hydroxybenzotriazole) afin d'obtenir l'ester activé de N-hydroxysuccinimide (éventuellement N-hydroxybenzotriazole) qui est également isolé.

L'étape d'acylation qui suit afin d'obtenir un composé de formule (III) s'effectue en présence d'une base selon les méthodes connues de l'homme du métier et est illustrée dans les exemples décrits plus loin. Il est également possible d'effectuer l'acylation sans isoler l'ester activé et sans base ajoutée en opérant en présence de chlorure de 4-(4,6-diméthoxy-1,3,5-triazin-2-yl)-4-myl-morpholinium (DMTMM).
Cette dernière alternative présente l'intérêt de supprimer une étape d'isolement et donc d'améliorer les rendements.

L'étape de déshydratation du composé de formule (III) afin d'obtenir un composé de formule (IV) peut s'effectuer de la manière suivante :
- L'étape de déshydratation s'effectue en présence d'un acide hydrohalogéné tel que HBr en présence de MgI₂. Dans ce cas, il n'est pas nécessaire de passer par l'intermédiaire de formule (III').
- A titre de variante, il peut être souhaitable d'alkyler le groupement hydroxyle en position 5 avant de procéder à l'étape de déshydratation. Par exemple on peut méthyler avec du méthanol en présence de PPTS (Pyridinium Paratoluène Sulfonate) afin d'obtenir un composé de formule (III') avec Alk représentant un méthyle.
- Enfin, cette réaction de déshydratation peut également s'effectuer en présence de bromure d'alpha-acétoxy isobutyryle (BAIB) dans le dioxane. Dans ce dernier cas on opère soit sur le composé de formule (III') soit directement sur le composé de formule (III) en présence de MgI₂.

De préférence, le composé obtenu suite à la réaction de déshydratation est ensuite purifié par cristallisation dans un mélange DMF(diméthylformamide)/MeCN (acétonitile). On peut également utiliser les mélanges de solvants suivants : DMF/acétone et DMF/AcOEt (acétate d'éthyle).

La réaction d'amination réductrice des composés de formule (IV) afin d'obtenir l'échinocandine de formule (I) s'effectue selon les méthodes décrites dans la littérature et connues de l'homme du métier (Yamada K. et al. J. Chem. Soc. Perkin Trans I (1983) 265-270 ; Hutchins R. et al. Org. prep. Proc. Int. 11(5) (1979) 201-246). En particulier on utilise l'éthylènediamine en présence de NaBH₃CN en couple avec le TiCl₄ ou tout autre acide de Lewis ou en présence de NaBH(OCOR'₃), OCOR' représentant la Boc-L-Pro, la Bzl-L-Pro ou tout autre aminoacide optiquement actif ainsi que tout autre acide carboxylique chiral ou non.

Le fait de coupler le réactif de réduction NaBH₃CN avec un acide de Lewis, notamment TiCl₄, est un élément essentiel pour la sélectivité et donc pour obtenir un mélange d'isomère A et d'isomère B dans lequel l'isomère actif A est majoritaire. En général on obtient un rapport en isomère A supérieur à 70%, et notamment compris entre 80 et 85%. Il en est de même avec les autres réactifs cités plus haut.

Notamment, pour le composé de formule (Ia), on obtient à ce stade un rapport isomère A/isomère B respectivement de 80-85 %/20-15 % alors que Le procédé tel que décrit dans WO 97/29716 mène à un mélange dans lequel l'isomère A est minoritaire ou équivalent (mélange racémique A et B).

Lors de l'étape d'amination réductrice, un intermédiaire réactionnel avant réduction peut le cas échéant, être isolé, et fait ainsi l'objet de la présente invention. Il s'agit d'une imidazolidine de formule (IV') ou (IV'a) lorsque R représente un groupement -Ph-Ph-O-C₈H₁₇

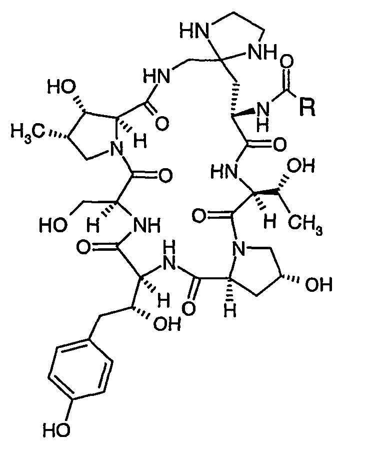

Il peut être souhaitable de purifier par chromatographie ou par recristallisation le ou les produits obtenus. Cette opération s'effectue selon les méthodes habituelles connues de l'homme du métier. Lorsque le produit obtenu est notamment sous forme de sel d'acide trifluoroacétique suite à une chromatographie, il est souhaitable d'éliminer ce sel par action d'une base afin d'obtenir le composé de formule (I) sous forme de base.

La réaction de salification s'effectue selon les méthodes usuelles connues de l'homme du métier. La préparation du chlorhydrate ou du di-chlorhydrate s'effectue en présence d'acide chlorhydrique dans le méthanol.

Tout particulièrement, la présente invention a pour objet un procédé de préparation des composés de formule (Ia) 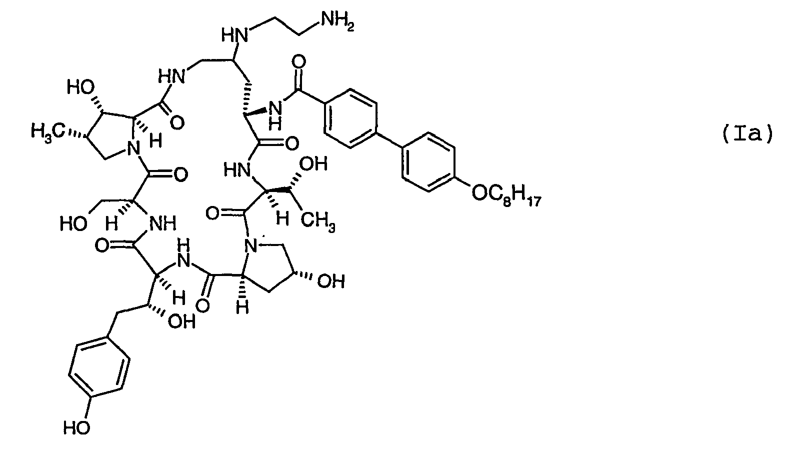 comprenant les étapes suivantes
a) On soumet un composé de formule (II) 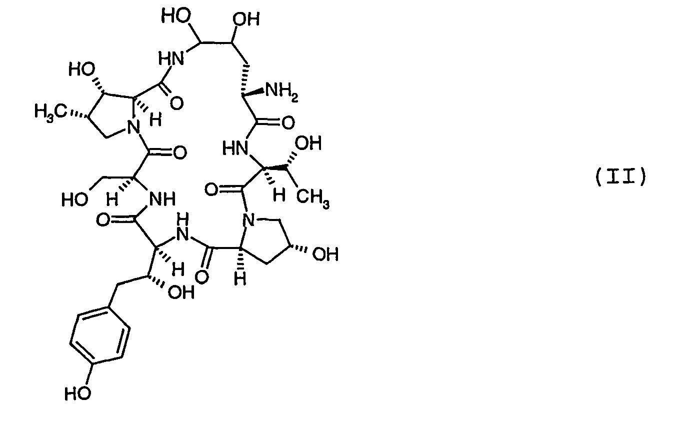 à l'action d'un acide de formule C₈H₁₇-O-Ph-Ph-CO₂H, le dit acide étant le cas échéant sous une forme activée, isolée ou non isolée, afin d'obtenir le composé de formule (IIIa) 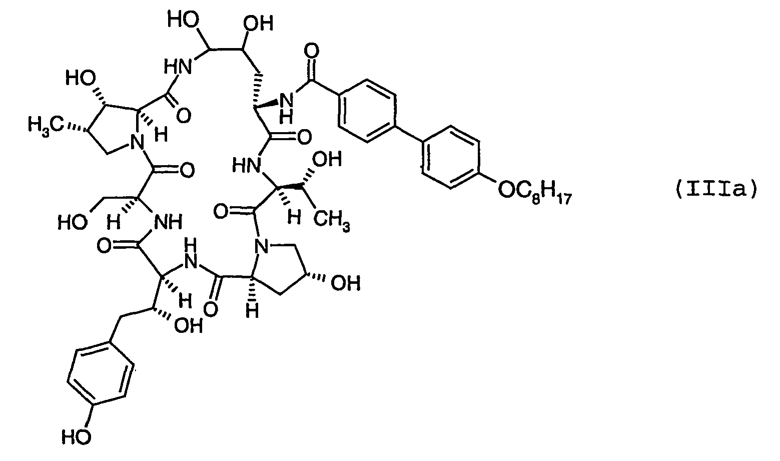
b) on soumet le composé de formule (IIIa) le cas échéant à une réaction d'alkylation de l'alcool en position 5 par action du méthanol en présence de PPTS pour obtenir le composé de formule (IIIa') 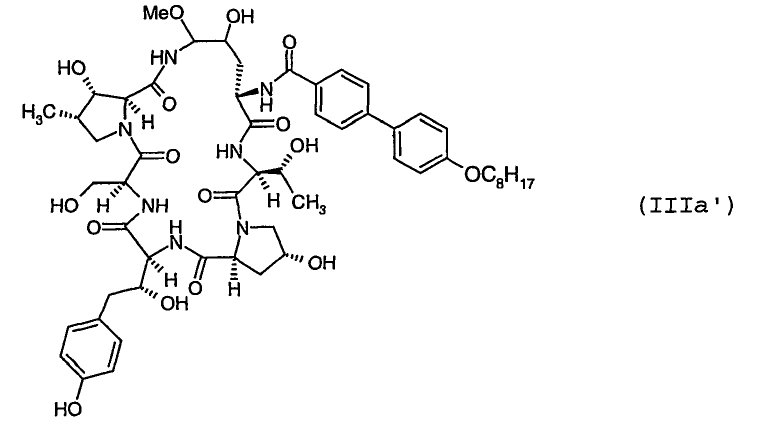
c) On soumet le composé de formule (IIIa) ou (IIIa') à une réaction de déshydratation, afin d'obtenir un composé de formule (IVa), 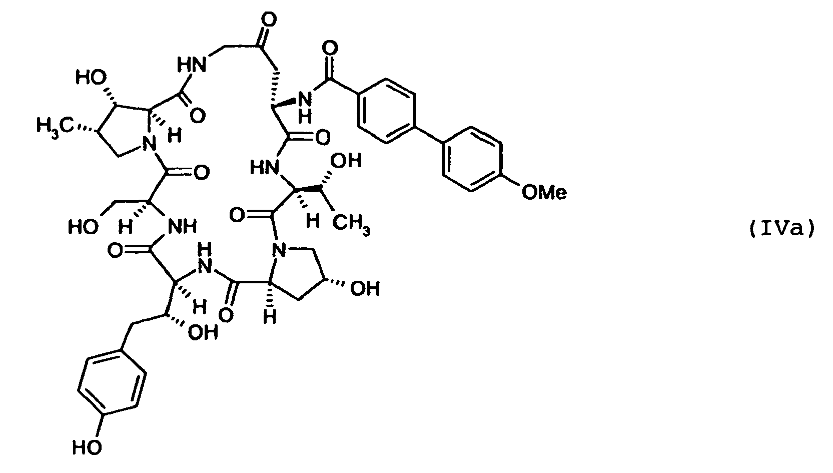
d) on soumet le composé de formule (IVa) à une réaction d'amination réductrice par action d'éthylène diamine en présence d'un agent réducteur tel que NaBH₃CN couplé avec le TiCl₄, le NaBH(Boc-L-Pro)₃ ou NaBH(Bzl-L-Pro)₃ afin d'obtenir le composé de formule (Ia) tel que défini plus haut, comportant majoritairement l'un des isomères actifs, le dit composé de formule (Ia) étant soumis à l'une ou plusieurs des opérations suivantes dans un ordre approprié

- chromatographie
- cristallisation
- action d'une base
- salification par action d'acide chlorhydrique.

Le composé de formule (Ia) peut se trouver sous la forme d'un mélange de deux épimères (Carbone asymétrique en position 4) que l'on appelle isomère A ou isomère B. Le composé de formule (I) obtenu selon le procédé tel que défini plus haut permet d'obtenir un mélange d'isomère A/isomère B d'environ 70-85 %/30-15 %.

Les étapes de purification/cristallisation et salifications qui sont effectuées suite à la réaction d'amination réductrice permettent d'obtenir l'épimère actif A du composé de formule (Ia) de façon sélective.

De préférence, le composé de formule (Ia) est purifié par chromatographie sur silice puis par chromatographie en phase inverse en utilisant un mélange de solvants organiques, d'eau et d'acide trifluoroacétique afin d'obtenir le sel d'acide trifluoroacétique du composé de formule (Ia). Ce sel est ensuite soumis à l'action d'une base, par exemple par action d'une solution aqueuse de bicarbonate de sodium, afin d'obtenir le composé de formule (Ia) sous forme de base. Puis, la base obtenue est salifiée par action d'acide chlorhydrique afin d'obtenir le sel correspondant à savoir le dichlorhydrate du composé de formule (Ia).

L'invention a donc tout particulièrement pour objet un procédé tel que défini précédemment caractérisé en ce que le composé de formule (Ia) subit successivement les opérations suivantes :
a) purification par chromatographie sur silice puis par chromatographie en phase inverse en utilisant un mélange de solvants organiques, d'eau et d'acide trifluoroacétique afin d'obtenir le sel d'acide trifluoroacétique du composé de formule (Ia)
b) action d'une base, par exemple par action d'une solution aqueuse de bicarbonate de sodium, afin d'obtenir le composé de formule (Ia) sous forme de base
c) salification par action d'acide chlorhydrique afin d'obtenir le sel correspondant à savoir le dichlorhydrate du composé de formule (Ia).

L'invention a donc tout particulièrement pour objet un procédé tel que défini précédemment caractérisé en ce que la réaction d'activation de l'acide s'effectue en présence de pentafluorophénol, de N-hydroxysuccinimide ou éventuellement de N-hydroxybenzotriazole.

L'invention a donc tout particulièrement pour objet un procédé tel que défini précédemment caractérisé en ce que la réaction d'acylation en présence d'un acide activé isolé, s'effectue en présence de diisopropyléthylamine.

L'invention a donc tout particulièrement pour objet un procédé tel que défini précédemment caractérisé en ce que la réaction d'activation puis d'acylation en présence d'un acide activé non isolé, s'effectue en présence de N-méthyl pyrrolidone et de DMTMM

L'invention a donc tout particulièrement pour objet un procédé tel que défini précédemment caractérisé en ce que la réaction de déshydratation s'effectue en présence de BAIB et le cas échéant de MgI₂.

L'invention a donc tout particulièrement pour objet un procédé tel que défini précédemment caractérisé en ce que la réaction de déshydratation s'effectue en présence de HBr-AcOH et de MgI₂.

L'invention a donc tout particulièrement pour objet un procédé tel que défini précédemment caractérisé en ce que le produit issu de la déshydratation est purifié par cristallisation dans un mélange diméthylformamide (DMF)/acétonitrile, DMF/acétone ou DMF/AcOEt.

L'invention a donc tout particulièrement pour objet un procédé tel que défini précédemment caractérisé en ce que la réaction d'amination réductrice s'effectue en présence d'agent réducteur choisi parmi NaBH₃CN couplé avec le TiCl₄, NaBH (Boc-L-Pro) ₃ et NaBH(Bzl-L-Pro)₃.
Le procédé de l'invention permet notamment de préparer le dichlorhydrate de 1-[4-[(2-aminoéthyl)amino]-N2-[[4'-(octyloxy)[1,1'-biphényl]-4-yl]carbonyl]-L-ornithine]-4-[4-(4-hydroxyphényl)-L-thréonine]-5-L-sérine échinocandine B sous forme 4S ou 4R ou sous forme d'un mélange des deux stéréoisomères.

Le procédé permet notamment d'obtenir l'isomère A du composé de formule (I) ou (Ia) de façon majoritaire avant séparation des isomères par chromatographie.
Le 1-[4-[(2-aminoéthyl)amino]-N2-[[4'-(octyloxy)-[1,1'-biphényl]-4-yl]carbonyl]-L-ornithine]-4-[4-(4-hydroxyphényl)-L-thréonine]-5-L-sérine échinocandine B est préparé dans WO 99/29716 sous la forme de base ou de sel d'acide trifluoroacétique (exemple 14). Le dichlorhydrate obtenu selon l'invention présente l'avantage de posséder une meilleure stabilité et solubilité dans l'eau.

### Exemple 1

### Dichlorhydrate de 1-[4-[(2-aminoyl)amino]-N2-[[4'-(octyloxy) [1,1'-biphényl]-4-yl]carbonyl]-L-ornithine]-4-[4-(4-hydroxyphényl)-L-thréonine]-5-L-sérine échinocandine B

### I) : Premier stade: Acylation du composé de formule (IIa) et obtention d'un composé de formule (III)

### 1-[(4R,5R)-4,5-dihydroxy-N2-[[4'-(octyloxy)[1,1'-biphényl]-4-yl]carbonyl]-L-ornithine]-4-[4-(4-hydroxyphényl)-L-thréonine]-5-L-sérine échinocandine B

### Voie 1: par l'ester activé de pentaflurophénol isolé

### Etape 1 : Préparation de l'ester activé

### 4'-Octyloxy-biphényl-4-carboxylate de pentafluorophényle

On introduit 100 g d'acide 4-octyloxybiphényl-4'carboxylique et 62 g de pentafluorophénol dans 1 litre de dichlorométhane. On agite 15 minutes et on ajoute en 15 minutes 69.6 g de N,N'-dicyclohexylcarbodiimide en solution dans 500 ml de dichlorométhane. La suspension beige foncée est agitée 20 h à température ambiante. On filtre la dicyclohexylurée et le filtrat est concentré. On distille à volume constant par rajout régulier d'éthanol jusqu'à température vapeur de 74 °C. On refroidit à température ambiante, agite une heure, filtre puis lave à l'éthanol.
Après séchage, on obtient 145.7 g de produit recherché sous forme de cristaux.
Rdt 96.5 %
RMN (CDCl₃) : 8.23-7.72 (AA'BB') 4H ; 7.01-7.60 (AA'BB') 4H ; 4.02 (t) 2H ; 1.82 (quint) 2H ; 1.48 (quint) 2H ; 1.32 (m) 8H ; 0.89 (t) 3H.

### Etape 2 : Acylation

On introduit 23.2 g de composé de formule (II) ("nucleus de deoxymuluncandine" préparé selon la préparation 2 de WO 99/29716) et 14.1 g d'ester obtenu plus haut dans 60 ml de DMF. On introduit à la suspension, 6.7 ml de diisopropyléthylamine et agite pendant 24 h sous atmosphère d'azote à température ambiante. On coule le milieu réactionnel homogène en 10 minutes dans un litre d'eau en agitant modérément, agite la suspension pendant 2 heures, filtre et lave le solide avec de l'eau. Le solide est séché sous vide à température ordinaire puis on le porte à reflux dans 100 ml de chlorure de méthylène, sous azote en agitant pendant 2 heures (40°C). On refroidit à température ambiante en 1 heure, agite 1 heure, filtre le solide et le rince trois fois au chlorure de méthylène. On sèche sous vide à température ordinaire. On obtient 26.9 g de produit attendu sous forme de solide beige.
Rdt 91.5 %
CCM : Rf :0.13 Plaque de silice ; révélation UV 254 nm ; éluant : CH₂Cl₂-MeOH-eau : 86-13-1
RMN : (DMSO)
Thréonine : 8.16 (1H), 4.85 (1H), 4.41 (1H), 1.13 (3H) ; γHydroxyproline : 4.42 (1H), 1.92-2.28 (2H), 4.44 (1H), 3.86-3.70 (2H) ; βHydroxy homo tyrosine 7.36 (1H), 4.23 (1H), 4.20 (1H), 2.53-2.46 (2H), 6.98 (2H), 6.68 (2H) ;
Sérine : 7.40 (1H), 4.86 (1H), 3.66-3.60 (2H), βHydroxy γméthyl proline : 4.27 (1H), 4.03 (1H), 2.38 (1H), 0.99 (3H), 3.25-3.93 (2H), « ornithine » : 7.98 (1H), 5.16 (1H), 3.96 (1H), 8.54 (1H), 4.44 (1H), 1.98 (2H) ;
Aromatique et chaîne octyloxy : 7.98 (2H), 7.71 (2H), 7.68 (2H), 7.02 (2H), 4.00 (2H), 1.72 (2H), 1.41 (2H), 1.28 (2H), 1.25 (2H), 1.31 (2H), 1.27 (2H), 0.86 (3H).

### Voie 2 : par l'ester activé d'HOSu isolé

### Etape 1 : Synthèse de l'ester activé

### 4'-Octyloxy-biphényl-4-carboxylate de 2,5-dioxopyrrolidine

On introduit 9.3 g d'acide octyloxybiphényl, 93 ml de dichlorométhane, 3.8 g de N-hydroxysuccinimide, 6.3 g d'EDC et agite 3 h à température ambiante. On ajoute 6 ml d'eau, agite 10 min, décante et réextrait avec 45 ml de dichlorométhane. La phase organique est lavée à l'eau (3 fois 45 ml) et on sèche sur sulfate de sodium. On amène à sec sous vide et on obtient 12.05 g de produit attendu sous forme de cristaux.
Rdt : 99.9%
RMN : CDCl₃ : 8,2-7,65 (AA'BB') 4H ; 7,6-6, 97 (AA'BB') 4H ; 4,02 (t, 2H) ; 2,93 (large) 4H ; 1,83 (quint 2H); 1,45 (m) 2H ; 1,3 (m) 8H ; 0,9 (t) 3H.

### Etape 2 : Acylation

On dissout 1.43 g d'ester succinimidique tel que préparé plus haut dans 6 ml de DMF. On introduit 2.42 g de "nucleus de deoxymuluncandine" (préparé selon la préparation 2 de WO 99/29716) et 0.66 ml de diisopropyléthylamine. On agite la solution pendant 18 heures à température ambiante. On introduit 35 ml d'eau et agite 2 heures à température ambiante. On filtre et reprend le solide dans 30 ml d'eau sous agitation pendant 2 heures dans un réacteur. On filtre et rince à l'eau. On sèche le solide sous vide à température ambiante et on obtient 2.75 g de produit attendu sous forme de solide beige.
Rdt 98.2 %

### Voie 3 : Synthèse par activation directe au DMTMM

On dissout 2.8 g de de deoxymuluncandine (préparé selon la préparation 2 de WO 99/29716) dans 8.3 ml de N-myl pyrolidone. On ajoute 1.12 g d'acide octyloxybiphényl et 0.95 g de chlorure de 4-(4,6-Dimoxy-1,3,5-triazin-2-yl)-4-mylmorpholinium (DMTMM). Après 24 heures d'agitation à température ambiante, on verse le milieu réactionnel dans 133 ml d'eau sous agitation. On agite 20 minutes, filtre le solide et le rince 3 fois avec de l'eau (3 fois 7 ml). On séche sous vide à 40°C et on obtient 2.66 g de produit attendu 4 sous forme de solide beige.
Rdt 73.5 %

### II).Deuxième stade : déshydratation (Obtention d'un composé de formule (IVa)

### 1-[N2-[4'-(octyloxy)-[1,1'-biphényl]-4-yl]carbonyl]-4-oxo-L-ornithine]-4-[4-(4-hydroxyphényl)-L-threonine]-5-L-serine-échinocandine B

### Voie 1 : en passant par le composé de formule (III'a)

### Etape 1 : Alkylation

### 1-[(4R,5R)-4-hydroxy,5-moxy-N2-[[4'-(octyloxy)[1,1'-biphényl]-4-yl]carbonyl]-L-ornithine]-4-[4-(4-hydroxyphényl)-L-thréoninel-5-L-sérine échinocandine B (III'a)

On dissout 283 g de produit IIIa obtenu au premier stade dans 5.67 litres de méthanol. On ajoute en une seule fois en agitant 22.7 g de pyridinium p-toluène sulfonate et agite pendant 6 heures à reflux (42 °C) et 12 heures à température ambiante. On concentre sous vide à deux volumes résiduels puis ajoute 1.2 litres d'eau. On agite la suspension pendant 18 heures. On filtre le solide et rince deux fois à l'eau. On sèche en étuve sous vide à température ambiante. On obtient 270.5 g de produit attendu sous forme de poudre beige.
Rdt 94.2 %
RMN : (DMSO)
Thréonine : 8.11 (1H), 4.84 (1H), 4.41 (1H), 1.11 (3H) ; γHydroxyproline : 4.40 (1H), 1.91-2.27 (2H), 4.42 (1H), 3.67-3.87 (2H) ; βHydroxy homo tyrosine 7.32 (1H), 4.22 (1H), 4.18 (1H), 2.51-2.44 (2H), 6.96 (2H), 6.65 (2H), 9.03 (1H) ; Sérine : 7.36 (1H), 4.87 (1H), 3.58-3.63 (2H), βHydroxy γméthyl proline : 4.32 (1H), 4.05 (1H), 2.37 (1H), 1.00 (3H), 3.27-3.94 (2H), « ornithine » : 7.88 (1H), 4.94 (1H), 4.06 (1H), 8.50 (1H), 4.43 (1H), 3.16 (3H) ; 1.93-2.03 (2H) ; Aromatique et chaîne octyloxy : 7.97 (2H), 7.70 (2H), 7.65 (2H), 7.02 (2H), 4.00 (2H), 1.73 (2H), 1.43 (2H), 1.32 (2H), 1.27-1.28 (6H), 0.87 (3H).

### Etape 2 : deshydratation

On dissout 265.3 g de produit méthoxylé (III'a) obtenu plus haut dans 5.3 litres de dioxane. On ajoute en 20 minutes 71.8 ml de bromure d'α-acétoxyisobutyryle (BAIB). On agite le milieu pendant 7 heures. On ajoute 4.1 litres de solution aqueuse saturée de bicarbonate de sodium (NaHCO₃) en 15 minutes. On agite pendant 15 minutes et distille le dioxane sous vide à une température ne dépassant pas 30 °C. On ajoute 3.2 litres d'eau et agite pendant 15 heures à température ambiante. On filtre le solide et rince deux fois à l'eau. On sèche en étuve sous vide. On obtient 251.2 g de produit attendu sous forme de solide ocre.
Rendement 97.5% Le produit est cristallisé selon la préparation décrite plus bas.

### Voie 2 : à partir du produit (IIIa) obtenu au stade a par action de BAIB/MgI₂/dioxane

On met en suspension 23.26 g d'iodure de magnésium anhydre dans 500 ml de dioxane et agite 30 minutes. On introduit 12.25 ml de bromure d'α-acétoxyisobutyryle (BAIB) et agite la suspension ocre à température ambiante pendant 45 minutes. On ajoute en 1 heure une solution de 50 g de produit (IIIa) obtenu au premier stade, dissous dans 400 ml de dioxane et rince l'ampoule de coulée avec 25 ml de dioxane. On agite la suspension pendant 19 heures à température ambiante. On introduit en 30 minutes une solution de 5 g de bicarbonate de sodium dissous dans 50 ml d'eau (pH 5-6 de fin d'addition). On agite 2 heures. On distille sous vide jusqu'à 250 ml de volume résiduel à une température intérieure inférieure à 35 °C. On rétablit la pression atmosphérique à l'azote, et ajoute 200 ml de diméthylformamide. On distille sous vide jusqu'à 250 ml de volume résiduel et on coule cette solution à température ambiante dans 2.8 litres d'eau et rince avec du DMF. On agite pendant 1 heure. On filtre le solide et rince avec de l'eau. Le solide est séché pendant 24 heures sous vide à 30 °C. On obtient 46 g de produit attendu sous forme de solide beige.
Rdt : 98.6%

Le produit est ensuite cristallisé selon la méthode décrite plus bas.

### Voie 3 : à partir du produit IIIa obtenu au premier stade par action de HBr-AcOH/MgI₂/MEC

On dissout 10 g de produit obtenu au stade a) dans 180ml de méthyléthyl cétone (MEC). On introduit 4.65 g d'iodure de magnésium anhydre et 10 ml de MEC. On agite 35 min à température ambiante puis refroidit à 20 °C. on introduit 3 ml d'une solution de HBr 33% dans AcOH. On agite la suspension pendant 4 h à 20°C. On ajoute 10 ml de solution aqueuse de NaHCO₃ (bicarbonate de sodium) saturée. On agite 1 h à 20°C (dissolution). On coule la solution en 2 h dans 700 ml d'eau tout en distillant la MEC sous vide à 40°C. on distille jusqu'à 430 ml résiduels. On rajoute 100 ml d'eau et continue à distiller jusqu'à 430 ml résiduels. On répète encore deux fois l'opération. On ramène la suspension à température ambiante et agite une nuit. On filtre le solide et lave avec 3 fois 50 ml d'eau. On sèche sous vide à température ambiante. On obtient 9.3 g de produit attendu sous forme de poudre beige.
Le produit est purifié par cristallisation selon la méthode décrite plus bas
Rdt 94.5 % tq

### Cristallisation du produit de formule (IVa) obtenu plus haut (voie 1, 2 ou 3)

On dissout en 1 heure, sous agitation et à température ambiante, 10 g de 1-[N2-[4'-(octyloxy)-[1,1'-biphényl]-4-yl]carbonyl]-4-oxo-L-ornithine]-4-[4-(4-hydroxyphényl)-L-threonine]-5-L-serine-échinocandine B (obtenu plus haut, voie 1, 2 ou 3) dans 30 ml de diméthylformamide (DMF). On introduit 51 mg d'amorce (cristaux de produit obtenu selon les voies 1, 2 ou 3) et on agite 2 heures. On introduit ensuite régulièrement et en 2 heures 67 ml d'acétonitrile. A la fin de l'introduction, on agite encore pendant 19 heures. On filtre et rince avec 3 fois 10 ml d'acétonitrile on sèche sous vide à température ambiante. On obtient 7.10 g de produit attendu sous forme de cristaux blancs.
Rdt : 71 %
CCM : rf = 0.28
Gel de silice 60F₂₅₄, UV 254 nm
Phase mobile : CH₂Cl₂/MeOH/eau 86/13/1
RMN : (DMSO)
Thréonine : 7.94 (1H), 4.50 (1H), 4.25 (1H), 1.18 (3H) ; γHydroxyproline : 4.39 (1H), 1.93-2.20 (2H), 4.40 (1H), 3.85-3.71 (2H) ; βHydroxy homo tyrosine 7.53 (1H), 4.06 (1H), 4.25 (1H), 2.44-2.53 (2H), 6.96 (2H), 6.66 (2H), 9.06 (1H) ;
Sérine . 7.74 (1H), 4.96 (1H), 3.72 (2H), βHydroxy γméthyl prolixe 4.33 (1H), 3.98 (1H), 2.31 (1H), 0.99 (3H), 3.26-4.0 (2H), « ornithine » : 8.37 (1H), 3.84-3.64 (2H), 8.11 (1H), 4.83 (1H), 2.82-3.11 (2H) ; Aromatique et chaîne octyloxy : 7.91 (2H), 7.69 (2H), 7.66 (2H), 7.02 (2H), 4.02 (2H), 1.74 (2H), 1.43 (2H), 1.32-1.28 (6H), 1.28, (2H) , 0.87 (3H).

### III : Troisième stade :Amination réductrice du composé de formule (IVa) et obtention d'un composé de formule (Ia)

### 1-[4-[(2-aminoyl)amino]-N2-[[4'-(octyloxy)[1,1'-biphényl]-4-yl]carbonyl]-L-ornithine]-4-[4-(4-hydroxyphényl)-L-thréonine]-5-L-sérine échinocandine B

### Voie 1 :

On dissout 8 g de produit de formule (IVa) obtenu selon le stade II et 2.52 ml d'éthylène diamine dans 80 ml de tetrahydrofurane (THF) en agitant de 30 minutes à 1 heure sous azote et à température ambiante. On ajoute en 30 minutes une solution de 0.36 g de tétrachlorure de Titane dissous dans 80 ml de THF. On agite 1 heure puis on refroidit à 5 °C. On ajoute 3.2 ml d'acide acétique glacial dissous dans 16 ml de THF. On agite 1 h à 5°C, refroidit à 0°C et on ajoute en 15 minutes une solution de 1.04 g de cyanoborohydrure de sodium dissous dans 24 ml de THF. On laisse remonter à 5°C en 30 minutes et on agite 30 minutes à cette température. Puis on laisse remonter à température ambiante en agitant 3 heures. On distille sous vide à 40 ml de volume, rajoute une solution de 7.2g de bicarbonate de sodium dissous dans 80 ml d'eau, distille sous vide jusqu'à 80 ml de volume résiduel puis ajoute 32 ml de méthanol et 128 ml d'acétate d'éthyle. On agite 10 minutes, décante, reextrait la phase aqueuse avec un mélange acétate d'éthyle / méthanol et distille l'ensemble des phases organiques sous vide jusqu'à 40 ml de volume résiduel. On introduit 40 ml de DMF et continue de distiller jusqu'à 60 ml de volume résiduel. On coule cette solution en 20 minutes dans 320 ml d'eau, agite 15 heures à température ambiante. On ajuste si nécessaire le pH à 9-9.5 avec de la soude diluée, filtre et rince avec une solution aqueuse de 1.2 g de bicarbonate de sodium (pH 9-9.5). On sèche le solide en étuve sous vide à 30°C pendant 18 heures et obtient 8.55 g de base brute attendue sous forme de solide blanc.
Rdt : 100 %
Ratio isomérique A/B = 80/20

### Voie 2 :

On introduit sous agitation et atmosphère d'azote 10 g de produit de formule (IV) obtenu au stade II) dans 600 ml de THF. On ajoute 20 g de Tamis moléculaire 3 Å puis 3.16 ml d'éthylène diamine, et enfin 110.4 g de Tri-benzyl-L-Proline-borohydrure de sodium. On agite à température ambiante la solution homogène pendant 6 heures. On filtre le tamis moléculaire et rince au THF. On distille le filtrat à sec sous vide à température intérieure inférieure à 40°C. On ajoute lentement sur la résine obtenue et en agitant, 600 ml de solution aqueuse saturée de bicarbonate de sodium. On ajoute sur la suspension 20 g d'adjuvant de filtration Hyflosupercel Kieselgühr et on agite 16 h à température ambiante. On filtre et lave à l'eau. On dissout le gâteau en faisant passer quatre fois 100 ml de méthanol. On concentre le filtrat méthanolique à sec sous vide sans dépasser 40°C. On obtient 21.25 g de produit attendu.

### Voie 3

### Isolement de l'intermédiaire de la réaction d'amination réductrice (composé de formule IV'a)

### 1-[4-[N,N'-imidazolidine]-N2-[[4'-(octyloxy)[1,1'-biphényl]-4-yl]carbonyl]-L-ornithine]-4-[4-(4-hydroxyphényl)-L-thréonine]-5-L-sérine échinocandine B

On agite une suspension de produit de formule (IVa) obtenu au stade II) dans 40 ml de dichlorométhane et 0.32 ml d'éthylène diamine pendant 18 heures. On rajoute 80 ml d'éther diéthylique et on agite pendant 5 heures. On filtre le solide et rince à l'éther diéthylique plusieurs fois. On sèche en étuve sous vide pendant 15 heures à température ambiante. On obtient 076 g de composé attendu sous forme d'un solide blanc. Cet intermédiaire est ensuite soumis à l'action d'une réaction de réduction selon les voies 1 ou 2 précédentes. Ce composé isolé est nouveau et fait partie de la présente invention.
Rdt : 73 %
MASSE : (FAB) MH⁺ 1100 ; MNa⁺ : 1122
RMN : Thréonine : 8.51 (1H), 4.72 (1H), 4.38 (1H), 1.20 (3H) ; γHydroxyproline : 4.37 (1H), 1.90-2.22 (2H), 4.42 (1H), 3.88-3.66 (2H) ; βHydroxy homo tyrosine 7.46 (1H), 4.17 (1H), 4.17 (1H), 2.46 (2H), 6.96 (2H), 6.65 (2H), Sérine : 7.47 (1H), 4.84 (1H), 3.65-3,60 (2H), βHydroxy γméthyl proline : 4.22 (1H), 3.93 (1H), 2.29 (1H), 0.95 (3H), 3.22-3.90 (2H), « ornithine » : 7.45 (1H), 2.72-3.51 (2H), 9.55 (1H), 4.28 (1H), 1.63-2.12 (2H) ; Imidazolidine : 2.63-2.94 (2H) ; 2.78-3.02 (2H) ; Aromatique et chaîne octyloxy : 7.87-7,72 (2H, 2H), 7.66 (2H), 7.03 (2H), 4.02 (2H), 1.72 (2H), 1.27 1.32 1.43 (4 x 2H), 1.29 (2H), 0.88 (3H).

### IV)° : Quatrième stade : Purification par chromatographie du composé de formule (Ia) obtenu précédemment (selon les voie 1, 2 ou 3) et séparation des deux isomères A et B (A ou B correspondant aux stéréoisomères R ou S en position 4) Di-trifluoroacétate de 1-[4-[(2-aminoyl)amino]-N2-[[4'-(octyloxy)[1,1'-biphényl]-4-yl]carbonyl]-L-ornithine]-4-[4-(4-hydroxyphényl)-L-thréonine]-5-L-sérine échinocandine B

On conditionne une colonne Prochrom LC 200 avec 2.5 g de silice merck 11763 en utilisant comme phase mobile le mélange chlorure de méthylène (43)-acétonitrile (50)-méthanol (7)-eau (5)-acide trifluoroacétique (1). On dissous 24 g du composé obtenu plus haut (stade III, voie 1) dans 126 ml de mélange composé d'acétonitrile (50)-méthanol (7)-eau (5)-TFA (1). On filtre la solution et on ajoute 86 ml de chlorure de méthylène. On injecte cette solution sur la colonne. L'élution est réalisée avec un débit de 76 1/h et une pression de 14 bars. La détection est faite à 280 nm. On distille sous vide les fractions collectées à température extérieure inférieure à 40°C. On obtient 36.75 g de produit sous forme de solide di-sel de TFA.
Ratio isomérique A/B = 99.3/0.7

### V) : Cinquième stade : Purification par chromatographie du di-trifluoroacétate (élimination des autres sels)

On conditionne une colonne Prochrom LC 50 avec 300 g de silice phase inverse Daisogel SP 120 n°5/1502, en utilisant comme phase mobile le mélange eau (90)-acétonitrile (10)-acide trifluoroacétique (0.1). On dissous 36.75 g du sel obtenu plus haut à la première chromatographie dans le mélange composé de 57.3 ml d'eau (90)-acétonitrile (10)-acide trifluoroacétique (0.1) et de 26.8 ml d'acétonitrile pur. On filtre la solution et on injecte cette solution sur la colonne. L'élution est réalisée avec eau (90)-acétonitrile (10)-acide trifluoroacétique (0.1) dans un premier temps pour sortir les sels minéraux, puis ensuite avec eau (50)-acétonitrile (50)-acide trifluoroacétique (0.1) pour sortir le produit. On distille sous vide les fractions collectées à température extérieure inférieure à 40°C. La solution aqueuse limpide résultante est lyophilisée. On obtient 15.59 g de produit attendu sous forme de solide blanc cotonneux di-sel de TFA.
Ratio isomèrique A/B = 99.4/0.6
Rdt cumulé des deux chromatographies : 53.8 %
Rdt de l'amino réduction : 28.7 %

### VI) : Sixième stade : Retour à la base

### 1-[4-[(2-aminoyl)amino]-N2-[[4'-(octyloxy)[1,1'-biphényl]-4-yl]carbonyl]-L-ornithine]-4-[4-(4-hydroxyphényl)-L-thréonine]-5-L-sérine échinocandine B

On dissout 14.37 g de composé obtenu plus haut, sous forme sel di-trifluoroacétate lyophilisé, dans 143.7 ml d'eau . On agite 15 minutes sous azote et ajoute en 15 minutes à température ambiante 57.5 ml de solution aqueuse saturée de bicarbonate de sodium. On agite 17 heures. On ajoute 430 ml d'un mélange acétate d'éthyle/méthanol 8/2 et on agite 15 minutes. On décante la phase organique supérieure et on réextrait la phase aqueuse avec 288 ml du mélange acétate d'éthyle (8)-méthanol (2). On joint les phases organiques, les décante soigneusement, et on les distille à sec sous vide sans dépasser 35°C. On ajoute sur l'extrait sec 143.7 ml d'eau et agite la suspension pendant 30 minutes. On filtre et lave à fin de fluorures avec de l'eau. On sèche le solide sous vide à 40°C et on obtient 10.91 g de produit attendu sous forme de poudre blanche.
Rdt : 91.6 %
MICROANALYSE : (eau : 8.8%)

| % | théorie | trouvé |
|---|---|---|
| C | 61.020 | 55.3-55.1 |
| H | 07.224 | 7.5-7.4 |
| N | 11.437 | 10.3-10.3 |
| F | 0 | Non détecté |

### VII) : Septième stade : Salification (acide Chlorhydrique) Dichlorhydrate de 1-[4-[(2-aminoy1)amino]-N2-[[4'-(octyloxy) [1,1'-biphényl]-4-yl]carbonyl]-L-ornithine]-4-[4-(4-hydroxyphényl)-L-thréonine]-5-L-sérine échinocandine B

On dissout 10.53 g de composé obtenu au stade précédent dans 263 ml de méthanol en agitant 30 minutes à température ambiante. On filtre et rince avec 2 fois 31.6 ml de méthanol. On ajoute sous agitation et azote à température ambiante, 2.1ml d'acide chlorhydrique 36%. On agite 30 minutes. On distille à sec sous vide sans dépasser 35 °C. On reprend l'extrait sec avec 105.3 ml d'oxyde de diisopropyle. On agite la suspension obtenue pendant 2 heures. On filtre et on lave avec deux fois 21.1 ml d'oxyde de diisopropyle. On sèche le solide en étuve sous vide à 40°C. On obtient 10.52 g de produit attendu sous forme de poudre blanche.
Rdt : 93.7%
MICROANALYSE : (eau 5.75 %)

| % | théorie | trouvé |
|---|---|---|
| C | 57.2 | 54.3 |
| H | 6.95 | 7.4 |
| N | 10.73 | 9.8 |
| Cl | 6.03 | 5.95 |
| F | 0 | Non détecté |

### VIII : Huitième stade : Cristallisation du sel d'acide chlorhydrique

### Dichlorhydrate de 1-[4-[(2-aminoyl)amino]-N2-[[4'-(octyloxy)[1,1'-biphényl]-4-yl]carbonyl]-L-ornithine]-4-[4-(4-hydroxyphényl)-L-thréonine]-5-L-sérine échinocandine B

On dissout 2.75 g de composé obtenu selon l'étape VII dans un mélange de 235.4 ml d'acétonitrile et 14.8 ml d'eau déminéralisée, en chauffant au reflux (77.4°C). On agite 15 minutes à 77 °C puis on refroidit en 45 minutes à + 50°C. On amorce avec 41.25 mg de composé cristallisé. On refroidit régulièrement en 30 minutes à 20°C. On agite pendant 2 h 15 min, pour laisser la cristallisation se développer. On concentre sous vide la suspension à 55 ml de volume résiduel en laissant évoluer la température de 5°C à 20°C. On rétablit la pression ordinaire à l'azote et on refroidit à 0°C en 20 minutes. On agite à 0°C pendant 17 heures, filtre sous pression d'azote et rince avec 11 ml d'acétonitrile. On sèche le solide cristallisé en étuve sous vide et à 40°C pendant 24 heures. On obtient 2.29 g de produit attendu sous forme de solide cristallin blanc.
Rdt 83.3 %
HPLC : Tr = 5.8 (isomère A) ; Tr = 7.1 (isomère B)
Kromasil C18, 15 cm, 4.6 mm, 5 µm
Phase mobile : acétonitrile-eau-TFA 50/50/0.1
210 nm, 35°C, 1 ml/mn.
RMN : CDCl₃
9.07 (m) 1H ; 8.48 (d, j=8) 1H ; 8.00 - (d, j=8) 2H ; 7.96 (d, j=8.5) 2H ; 7.71 (d, j=8.5) 2H ; 7.64 (d, j=8.5) 2H ; 7.60 (d, j=9) 1H ; 7.37 (d, j=9.5) 1H ; 7.02 (d, j=8.5) 2H ; 6.97 (d, j=8.5) 2H ; 6.65 (d, j=8.5) 2H ; 4.90 (m) 1H ; 4.77 (m) 1H ; 4.66 (m) 1H ; 4.45 (m) 1H ; 4.42 (m) 1H ; 4.39 (m) 1H ; 4.34 (s) 1H ; 4.26 (m) 1H ; 4.22 (m) 1H ; 4.08 (m) 1H ; 4.01 (t, j=6.5) 2H ; 3.88 (m) 3H ; 3.70 (m) 21H ; 3.51 (m) 2H ; 3.48 (m) 1H ; 3.31 (m) 2H ; 3.28 (m) 1H ; 3.16 (m) 2H ; 2.53 (dd, j=6 et 13.5) 1H ; 2.44 (dd, j=7.5 et 13.5) 1H ; 2.27 (m) 1H ; 2.25 (m) 1H ; 2.15 (m) 2H ; 1.94 (m) 1H ; 1.74 (m) 2H ; 1.44 (m) 2H ; 1.22 à 1.40 (m) 8H ; 1.13 (d, j=6) 3H ; 0.99 (d, j=6.5) 3H ; 0.88 (t, j=7) 3H.

### Exemple 2 : Composition pharmaceutique

On a préparé des comprimés renfermant
- produit de l'exemple 1 150 mg
- Excipient q.s.P 1g
Détail de l'excipient : amidon, talc, stéarate de magnésium.

## Revendications

1. Procédé de préparation de composés de formule (I) 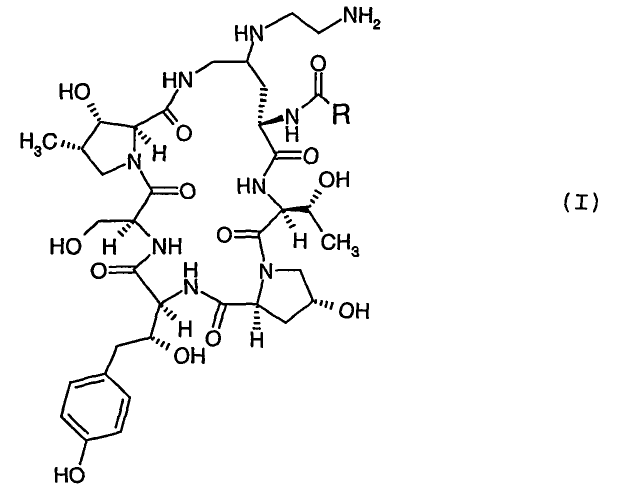 dans laquelle R représente une chaîne linéaire ou ramifiée ou cyclique renfermant jusqu'à 30 atomes de carbone, renfermant éventuellement un ou plusieurs hétéroatomes et un ou plusieurs hétérocycles
comprenant les étapes suivantes
a) On soumet un composé de formule (II) 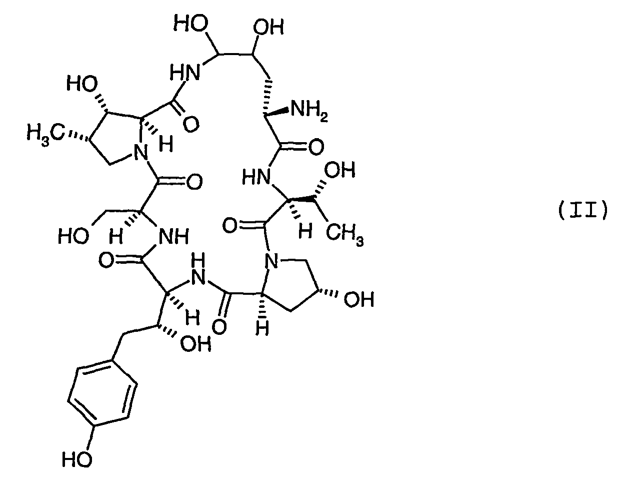 à l'action d'un acide de formule R-CO2H, le dit acide étant le cas échéant sous une forme activée, isolée ou non isolée, afin d'obtenir le composé de formule (III) 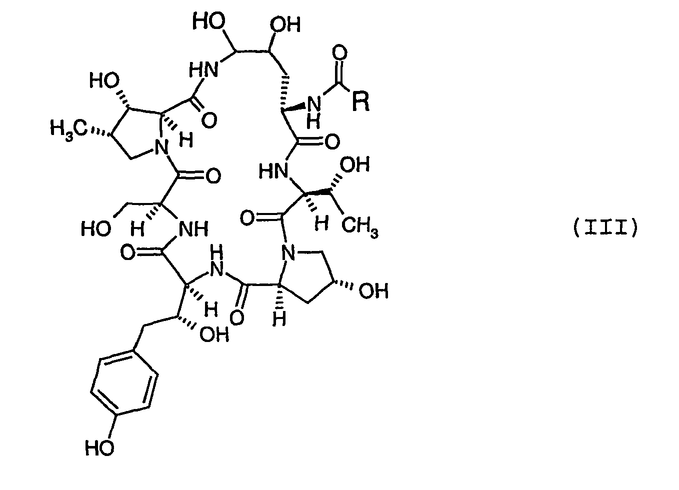
b) le cas échéant, on soumet le composé de formule (III) à une réaction d'alkylation de l'alcool en position 5 par action d'un alcool de formule Alk-OH en présence de PPTS, Alk représentant un akyle renfermant de 1 à 4 atomes de carbone pour obtenir le composé de formule (III') 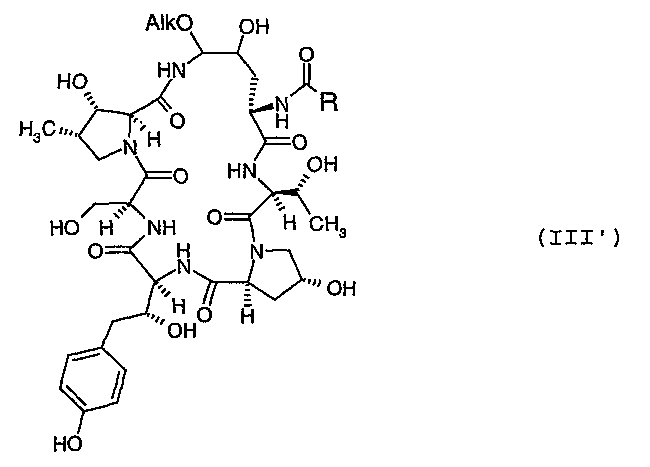
c) On soumet le composé de formule (III) ou (III') à une réaction de deshydratation, afin d'obtenir un composé de formule (IV) 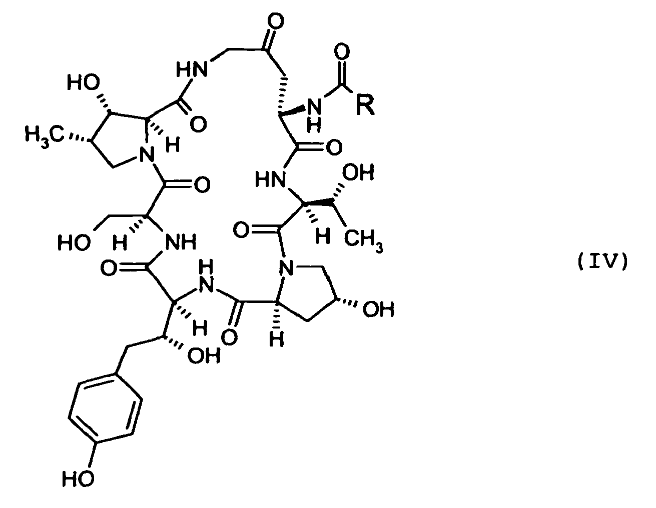
d) on soumet le composé de formule (IV) à une réaction d'amination réductrice par action d'éthylène diamine en présence d'un agent réducteur tel que NaBH₃CN en présence d'un acide de Lewis, ou le NaBH(OCOR')₃, OCOR' représentant la Boc-L-Pro, la Bzl-L-Pro ou tout autre aminoacide optiquement actif ainsi que tout autre acide carboxylique chiral ou non, afin d'obtenir le composé de formule (I) tel que défini plus haut, comportant majoritairement l'un des isomères actifs, le dit composé de formule (I) étant soumis à l'une ou plusieurs des opérations suivantes, dans un ordre approprié
- chromatographie
- cristallisation
- action d'une base
- salification.

2. Procédé tel que défini à la revendication 1 dans lequel les composés de formules (I), (III), (III') ou (IV) renferment un radical R représentant les groupements suivants :

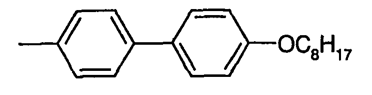

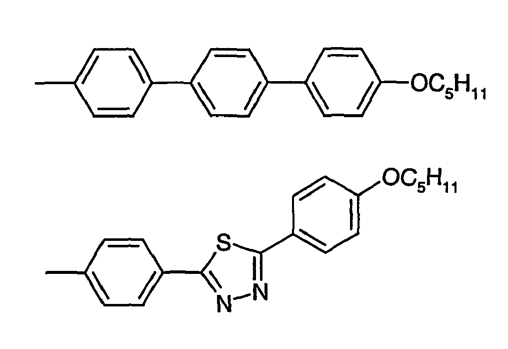

3. Procédé tel que défini à la revendication 1 dans lequel les composés de formules (I), (III), (III'), (IV) renferment un radical R représentant le groupement suivant :

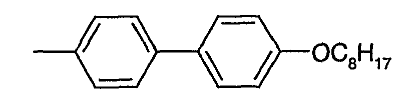

4. procédé de préparation de composés de formule (Ia) 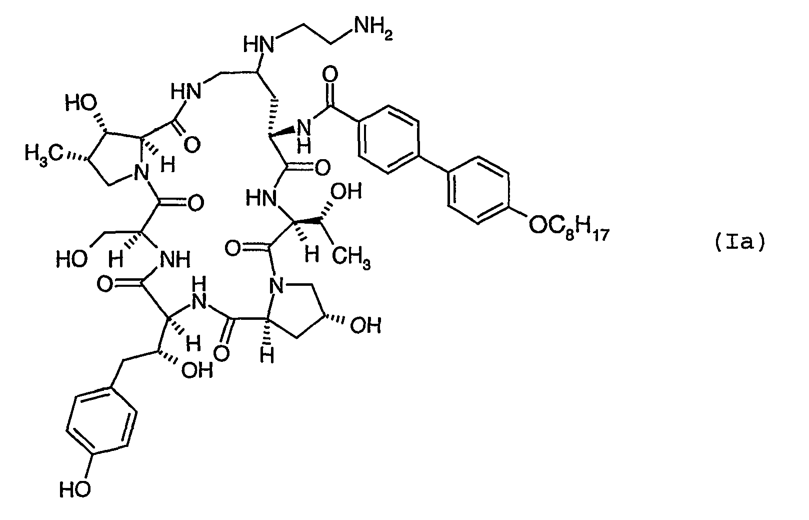 comprenant les étapes suivantes
a) On soumet un composé de formule (II) 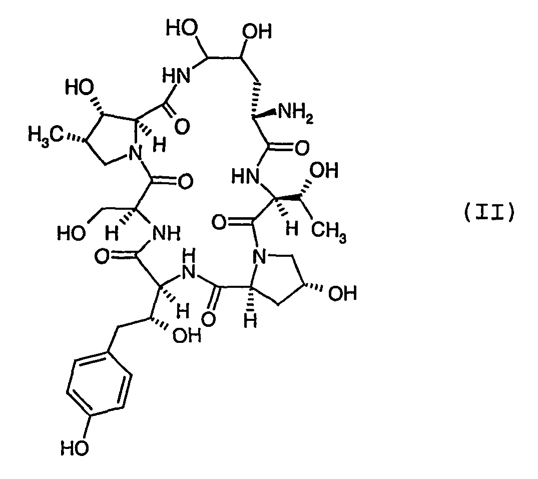 à l'action d'un acide de formule C₈H₁₇-O-Ph-Ph-CO₂H, le dit acide étant le cas échéant sous,une forme activée, isolée ou non isolée, afin d'obtenir le composé de formule (IIIa) 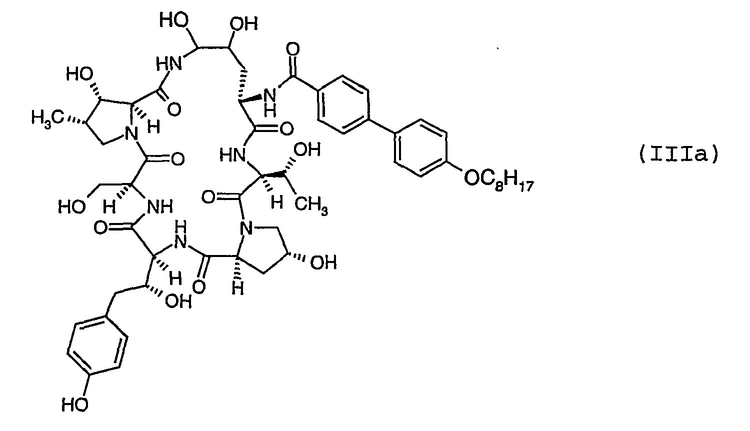
b) le cas échéant on soumet le composé de formule (IIIa) à une réaction d'alkylation de l'alcool en position 5 par action du méthanol en présence de PPTS pour obtenir le composé de formule (III'a) 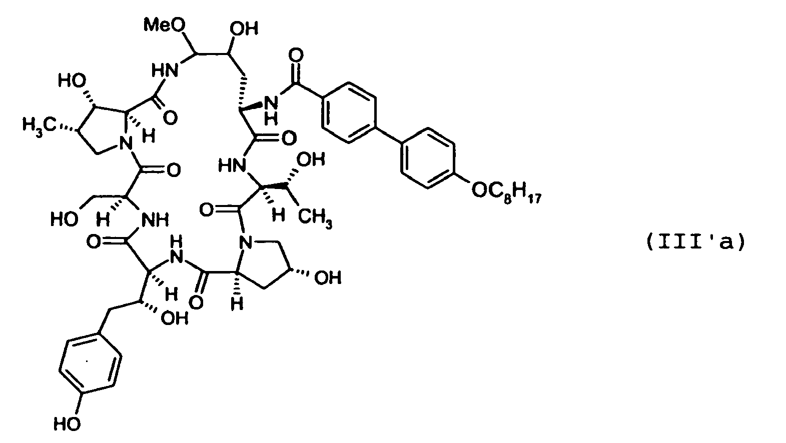
c) on soumet le composé de formule (IIIa) ou (III'a) à une réaction de deshydratation, afin d'obtenir un composé de formule (IVa) 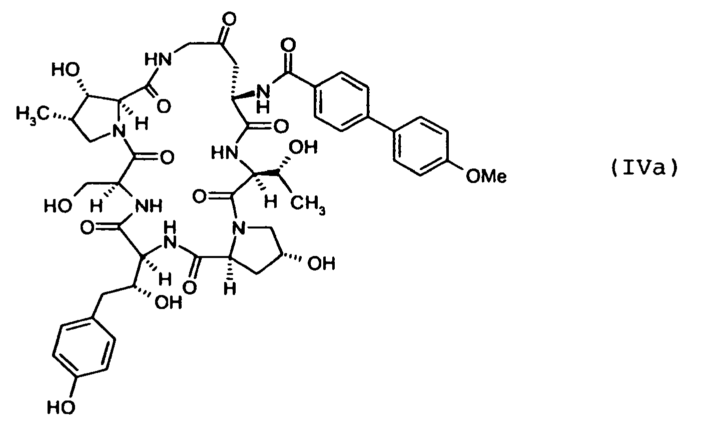
c) on soumet le composé de formule (IVa) à une réaction d'amination réductrice par action d'éthylène diamine en présence d'un agent réducteur tel que NaBH₃CN couplé avec le TiCl₄, le NaBH(Boc-L-Pro)₃ ou NaBH(Bzl-L-Pro)₃ afin d'obtenir le composé de formule (Ia) tel que défini plus haut, comportant majoritairement l'un des isomères actifs, le dit composé de formule (Ia) étant soumis à l'une ou plusieurs des opérations suivantes
- chromatographie
- cristallisation
- action d'une base
- salification par action d'acide chlorhydrique.

5. Procédé selon la revendication 4 **caractérisé en ce que** le composé de formule (Ia) subit successivement les opérations suivantes :
a) purification par chromatographie sur silice puis par chromatographie en phase inverse, en utilisant un mélange de solvants organiques, d'eau et d'acide trifluoroacétique afin d'obtenir le sel d'acide trifluoroacétique du composé de formule (Ia)
b) action d'une base, par exemple par action d'une solution aqueuse de bicarbonate de sodium, afin d'obtenir le composé de formule (Ia) sous forme de base
c) salification par action d'acide chlorhydrique afin d'obtenir le sel correspondant à savoir le dichlorhydrate du composé de formule (Ia).

6. Procédé selon l'une quelconque des revendications 1 à 5 **caractérisées en ce que** la réaction d'activation de l'acide s'effectue en présence de pentafluorophénol, de N-hydroxysuccinimide ou éventuellement de N-hydroxybenzotriazole.

7. Procédé selon l'une quelconque des revendications 1 à 5 **caractérisées en ce que** la réaction d'acylation en présence d'un acide activé isolé , s'effectue en présence de diisopropyléthylamine.

8. Procédé selon l'une quelconque des revendications 1 à 5 **caractérisées en ce que** la réaction d'activation puis d'acylation en présence d'un acide activé non isolé , s'effectue en présence de N-méthyl pyrrolidone et de DMTMM.

9. Procédé selon l'une quelconque des revendications 1 à 5 **caractérisées en ce que** la réaction de deshydratation s'effectue en présence de BAIB et le cas échéant de MgI₂.

10. Procédé selon l'une quelconque des revendications 1 à 7 **caractérisées en ce que** la réaction de déshydratation s'effectue en présence de HBr-AcOH et de MgI₂.

11. Procédé selon les revendications 9 et 10 **caractérisé en ce que** le produit issu de la déshydratation est purifié par cristallisation dans un mélange DMF/acétone ou DMF/AcOEt.

12. Procédé selon l'une quelconque des revendications 1 à 5 **caractérisées en ce que** la réaction d'amination réductrice s'effectue en présence d'agent réducteur choisi parmi NaBH₃CN couplé avec le TiCl₄ , NaBH(Boc-L-Pro)₃ et NaBH(Bzl-L-Pro)₃.

13. Procédé de préparation d'un composé de formule (IV) ou (IVa) par une réaction de déshydratation des composés de formules (III') ou (III'a) telle que définie à l'une quelconque des revendications 1 à 4 et 9 à 11.

14. Procédé de préparation d'un composé de formule (I) ou (Ia) par une réaction d'amination réductrice des composés de formules (IV) ou (IVa) telle que définie à l'une quelconque des revendications 1 à 4 et 12.

15. A titre de composés intermédiaires les composés de formules (III') ou (III'a) tels que définis aux revendications 1 et 4.

16. A titre de composés intermédiaires les composés de formules (IV'), R étant tel que défini à l'une quelconque des revendications 1 à 2, et (IV'a) lorsque R représente Ph-Ph-OC₈H₁₇

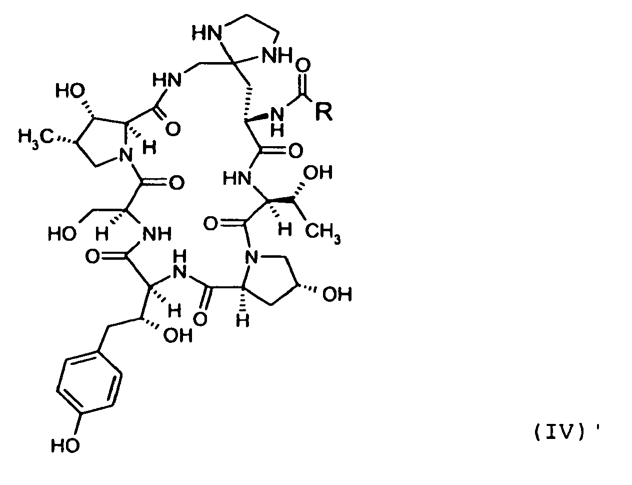

## Claims

1. Process for the preparation of compounds of formula (I): 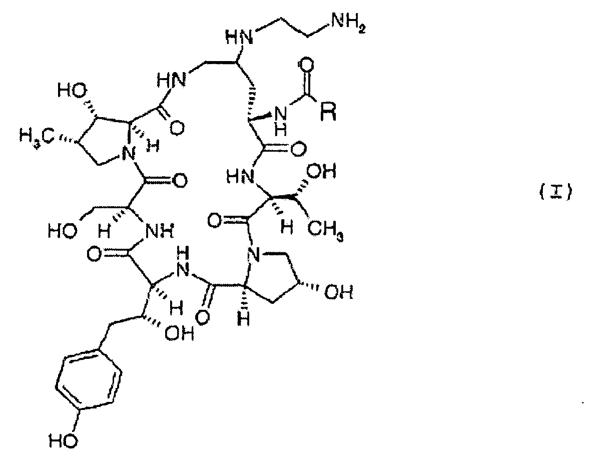 in which R represents a linear or branched or cyclic chain containing up to 30 carbon atoms, optionally containing one or more heteroatoms and one or more heterocycles
comprising the following stages
a) A compound of formula (II) 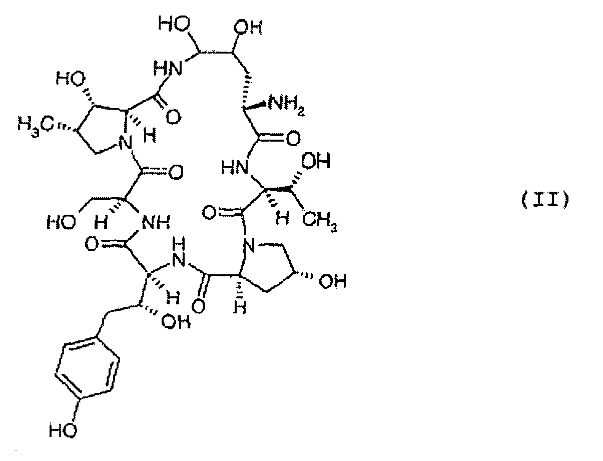 is subjected to the action of an acid of formula R-CO2H, said acid being, if appropriate, in an activated, isolated or non-isolated form, in order to obtain the compound of formula (III) 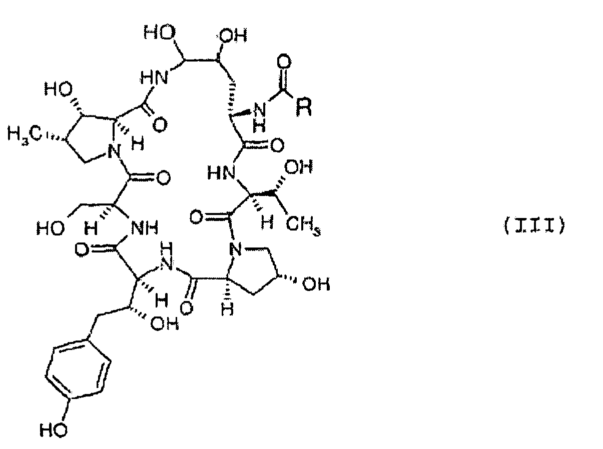
b) if appropriate, the compound of formula (III) is subjected to an alkylation reaction of the alcohol in position 5 by the action of an alcohol of formula Alk-OH in the presence of PPTS, Alk representing an alkyl containing 1 to 4 carbon atoms in order to obtain the compound of formula (III') 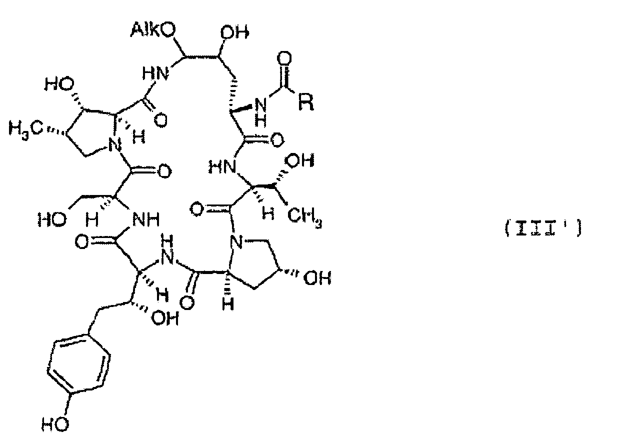
c) The compound of formula (III) or (III') is subjected to a dehydration reaction, in order to obtain a compound of formula (IV) 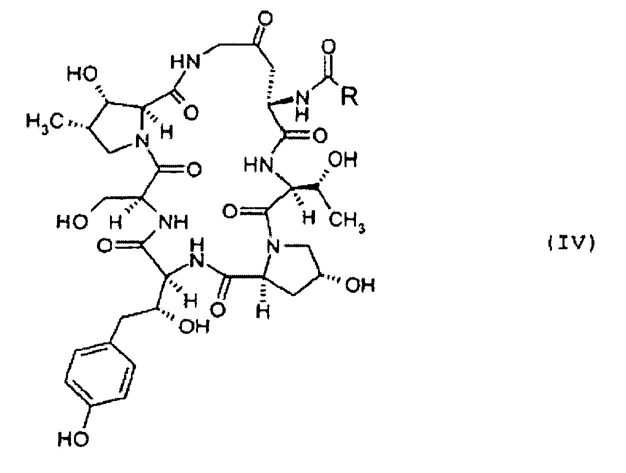
d) the compound of formula (IV) is subjected to a reducing amination reaction by the action of ethylene diamine in the presence of a reducing agent such as NaBH₃CN in the presence of a Lewis acid, or NaBH (OCOR')₃, OCOR' representing Boc-L-Pro, Bzl-L-Pro or any other optically active amino acid as well as any other chiral or non-chiral carboxylic acid, in order to obtain the compound of formula (I) as defined above, mostly comprising one of the active isomers, said compound of formula (I) being subjected to one or more of the following operations, in an appropriate order
- chromatography
- crystallization
- action of a base
- salification.

2. Process as defined in claim 1 in which the compounds of formulae (I), (III), (III') or (IV) contain an R radical representing the following groups:

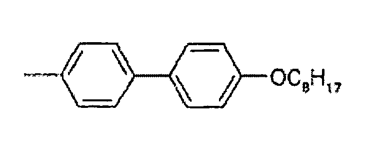

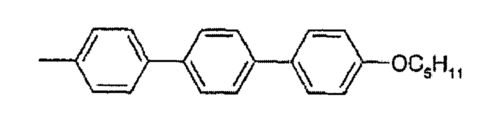

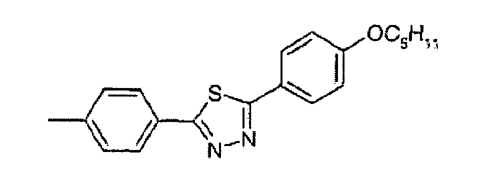

3. Process as defined in claim 1 in which the compounds of formulae (I), (III), (III'), (IV) contain an R radical representing the following group:

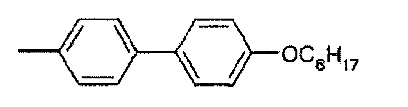

4. Process for the preparation of compounds of formula (Ia) 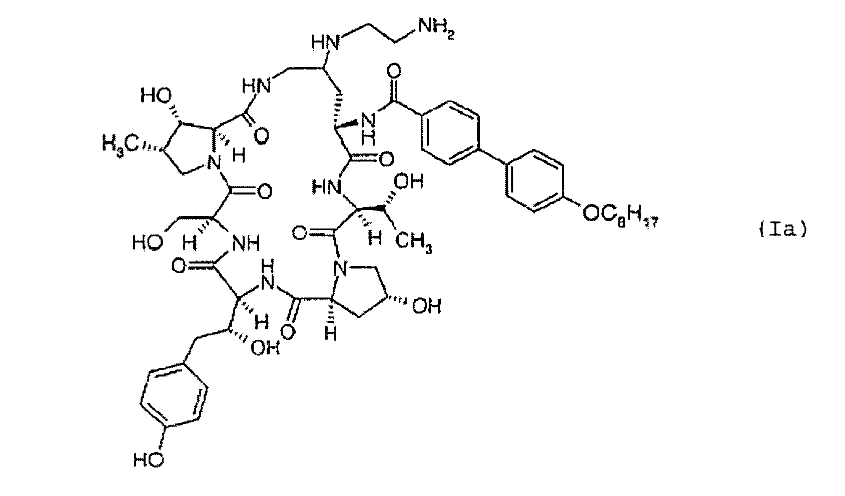 comprising the following stages
a) A compound of formula (II) 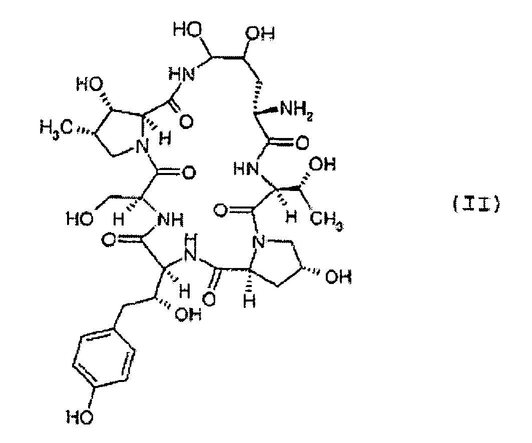 is subjected to the action of an acid of formula C₈H₁₇-O-Ph-Ph-CO₂H, said acid being, if appropriate, in an activated, isolated or non-isolated form, in order to obtain the compound of formula (IIIa) 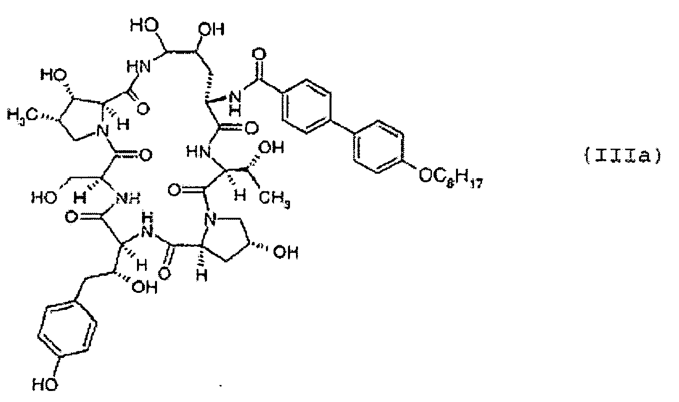
b) if appropriate, the compound of formula (IIIa) is subjected to an alkylation reaction of the alcohol in position 5 by the action of methanol in the presence of PPTS in order to obtain the compound of formula (III'a) 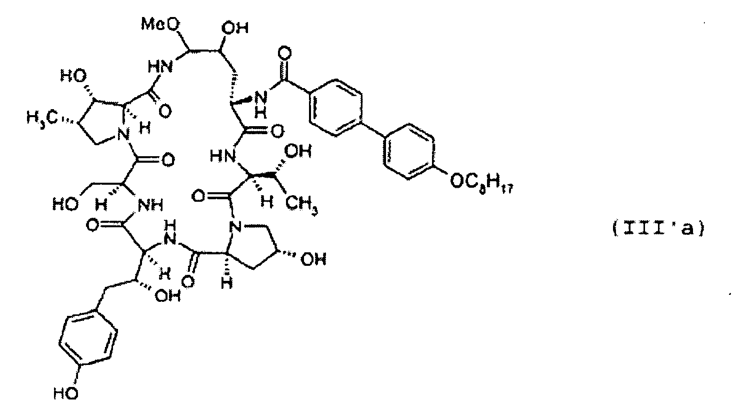
c) the compound of formula (IIIa) or (III'a) is subjected to a dehydration reaction, in order to obtain a compound of formula (IVa) 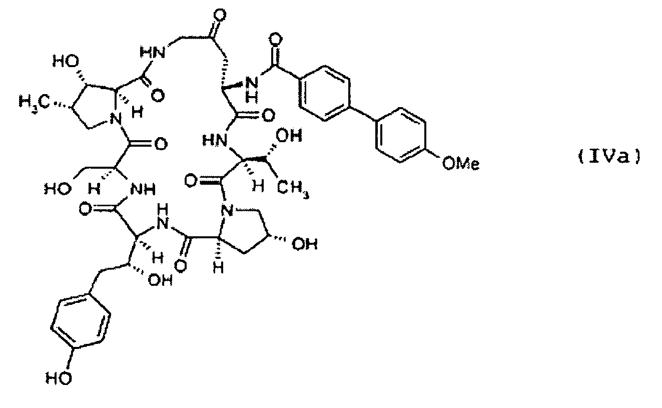
c) the compound of formula (IVa) is subjected to a reducing amination reaction by the action of ethylene diamine in the presence of a reducing agent such as NaBH₃CN coupled with TiCl₄, NaBH(Boc-L-pro)₃ or NaBH(Bzl-L-Pro)₃ in order to obtain the compound of formula (Ia) as defined above, mostly comprising one of the active isomers, said compound of formula (Ia) being subjected to one or more of the following operations
- chromatography
- crystallization
- action of a base
- salification by the action of hydrochloric acid.

5. Process according to claim 4 **characterized in that** the compound of formula (Ia) successively undergoes the following operations:
a) purification by chromatography on silica then by reversed-phase chromatography, using a mixture of organic solvents, water and trifluoroacetic acid in order to obtain the trifluoroacetic acid salt of the compound of formula (Ia)
b) action of a base, for example by the action of an aqueous solution of sodium bicarbonate, in order to obtain the compound of formula (Ia) in the form of base
c) salification by the action of hydrochloric acid in order to obtain the corresponding salt namely the dihydrochloride of the compound of formula (Ia).

6. Process according to any one of claims 1 to 5 **characterized in that** the activation reaction of the acid is carried out in the presence of pentafluorophenol, N-hydroxysuccinimide or optionally N-hydroxybenzotriazole.

7. Process according to any one of claims 1 to 5 **characterized in that** the acylation reaction in the presence of an isolated activated acid, is carried out in the presence of diisopropylethylamine.

8. Process according to any one of claims 1 to 5 **characterized in that** the activation then acylation reaction in the presence of a non-isolated activated acid, is carried out in the presence of N-methyl pyrrolidone and DMTMM.

9. Process according to any one of claims 1 to 5 **characterized in that** the dehydration reaction is carried out in the presence of BAIB and if appropriate of MgI₂

10. Process according to any one of claims 1 to 7 **characterized in that** the dehydration reaction is carried out in the presence of HBr-AcOH and MgI₂.

11. Process according to claims 9 and 10 **characterized in that** the product originating from the dehydration is purified by crystallization from a DMF/acetone or DMF/AcOEt mixture.

12. Process according to any one of claims 1 to 5 **characterized in that** the reducing amination reaction is carried out in the presence of a reducing agent chosen from NaBH₃CN coupled with TiCl₄, NaBH(BOC-L-Pro)₃ and NaBH(Bzl-L-Pro)₃.

13. Process for the preparation of a compound of formula (IV) or (IVa) by a dehydration reaction of the compounds of formulae (III') or (III'a) as defined in any one of claims 1 to 4 and 9 to 11.

14. Process for the preparation of a compound of formula (I) or (Ia) by a reducing amination reaction of the compounds of formulae (IV) or (Iva) as defined in any one of claims 1 to 4 and 12.

15. As intermediate compounds the compounds of formulae (III') or (III'a) as defined in claims 1 and 4.

16. As intermediate compounds the compounds of formulae (IV'), R being as defined in any one of claims 1 to 2, and (IV'a) when R represents Ph-Ph-OC₈H₁₇.

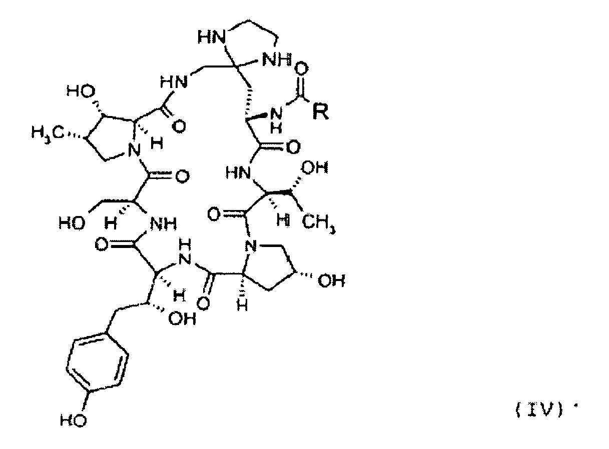

## Patentansprüche

1. Verfahren zur Herstellung von Verbindungen der folgenden Formel (I): 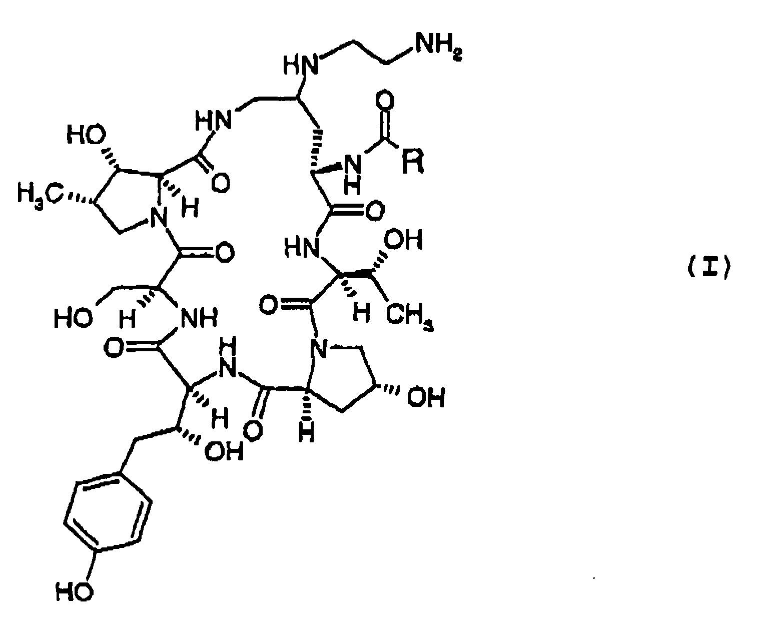 wobei R für eine lineare oder verzweigte oder cyclische Kette steht, die bis zu 30 Kohlenstoffatome enthält, die optional ein oder mehrere Heteroatome und ein oder mehrere Heterocyclen enthält,
das die folgenden Schritte umfasst:
a) Eine Verbindung der Formel (II) 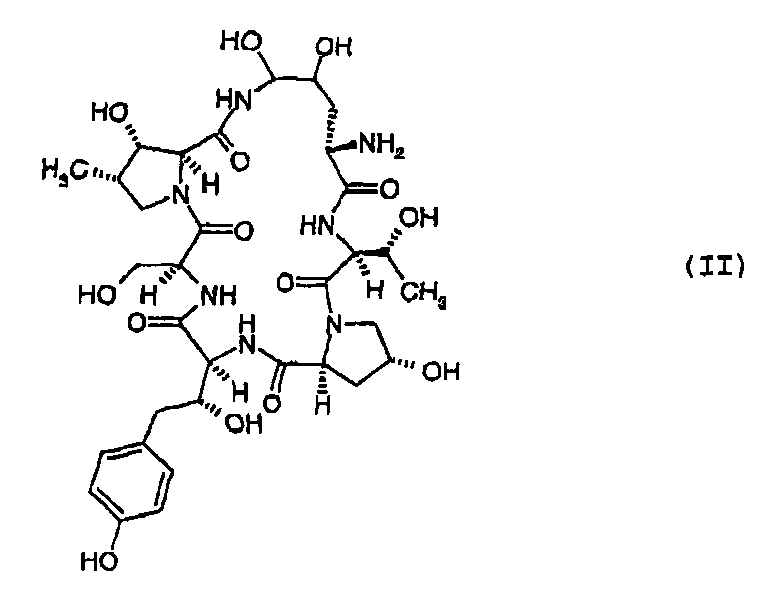 wird der Wirkung einer Säure der Formel R-CO₂H unterzogen, wobei die Säure gegebenenfalls in aktivierter, isolierter oder nicht isolierter Form vorliegt, um die Verbindung der folgenden Formel (III) zu erhalten: 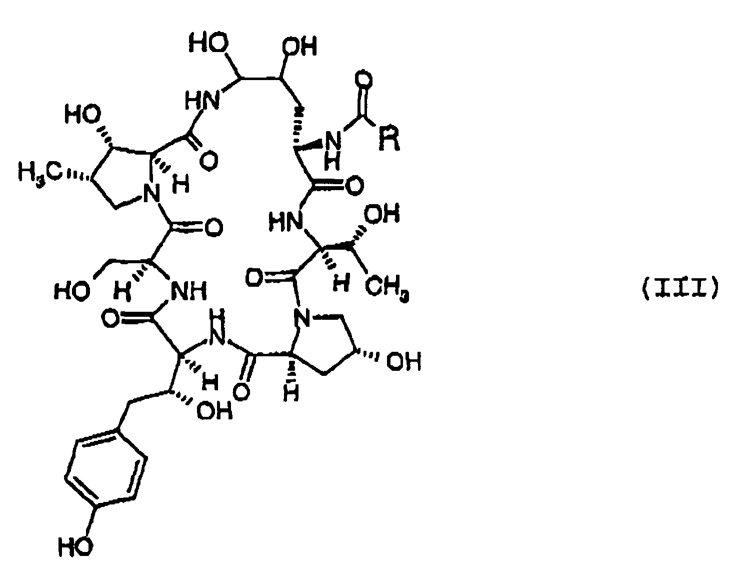
b) Die Verbindung der Formel (III) wird gegebenenfalls einer Alkylierungsreaktion des Alkohols an Position 5 durch die Wirkung eines Alkohols der Formel Alk-OH in Gegenwart von PPTS unterzogen, wobei Alk für ein Alkyl steht, das 1 bis 4 Kohlenstoffatome enthält, um die Verbindung der folgenden Formel (III') zu erhalten: 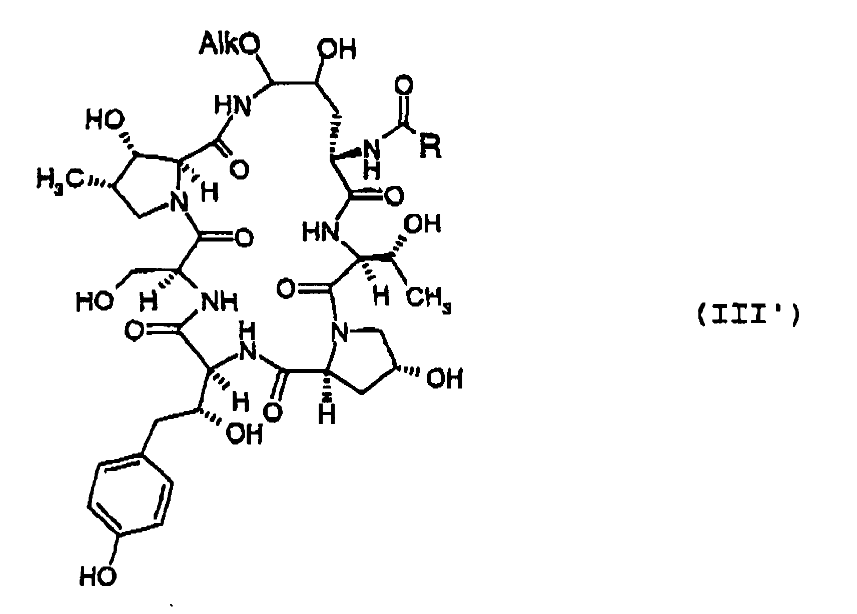
c) Die Verbindung der Formel (III) oder (III') wird einer Dehydrierungsreaktion unterzogen, um ein Verbindung der folgenden Formel (IV) zu erhalten: 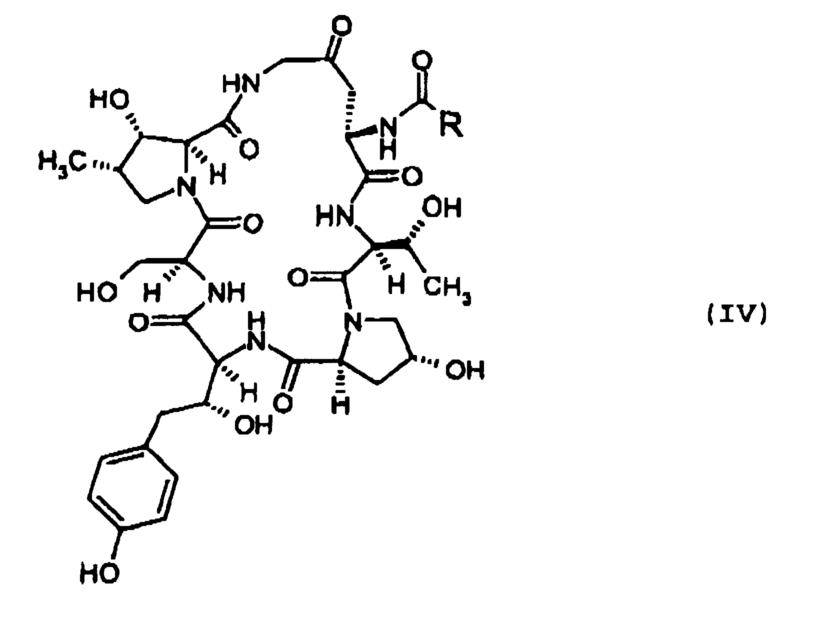
d) Die Verbindung der Formel (IV) wird einer reduzierenden Aminierungsreaktion durch die Wirkung von Ethylendiamin in Gegenwart eines Reduktionsmittels, wie etwa NaBH₃CN in Gegenwart einer Lewissäure oder NaBH(OCOR')₃ unterzogen, wobei OCOR' für Boc-L-Pro, Bzl-L-Pro oder jede andere optisch aktive Aminosäure sowie jede andere chirale oder nicht chirale Carbonsäure steht, um die Verbindung der Formel (I) zu erhalten, wie weiter oben definiert, die hauptsächlich eines der aktiven Isomere umfasst, wobei die Verbindung der Formel (I) einem oder mehreren der folgenden Arbeitsgänge in einer geeigneten Reihenfolge unterzogen wird:
- Chromatographie
- Kristallisation
- Wirkung einer Base
- Salzbildung.

2. Verfahren wie in Anspruch 1 definiert, wobei die Verbindungen der Formeln (I), (III), (III') oder (IV) einen Rest R enthalten, der für die folgenden Gruppen steht:

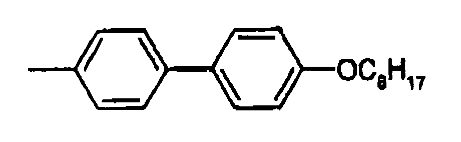

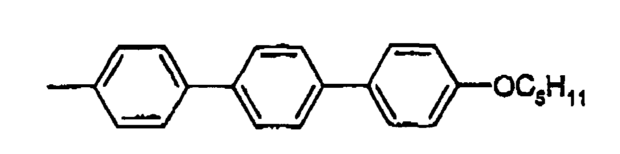

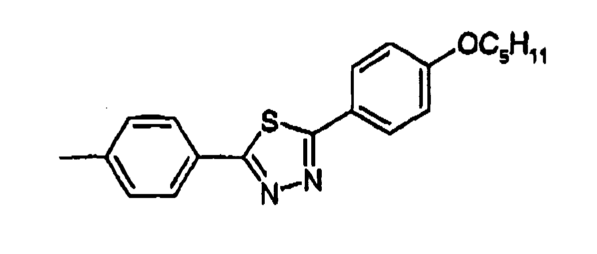

3. Verfahren wie in Anspruch 1 definiert, wobei die Verbindungen der Formeln (I), (III), (III'), (IV) einen Rest R enthalten, der für die folgende Gruppe steht:

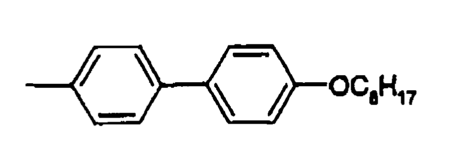

4. Verfahren zur Herstellung von Verbindungen der folgenden Formel (Ia): 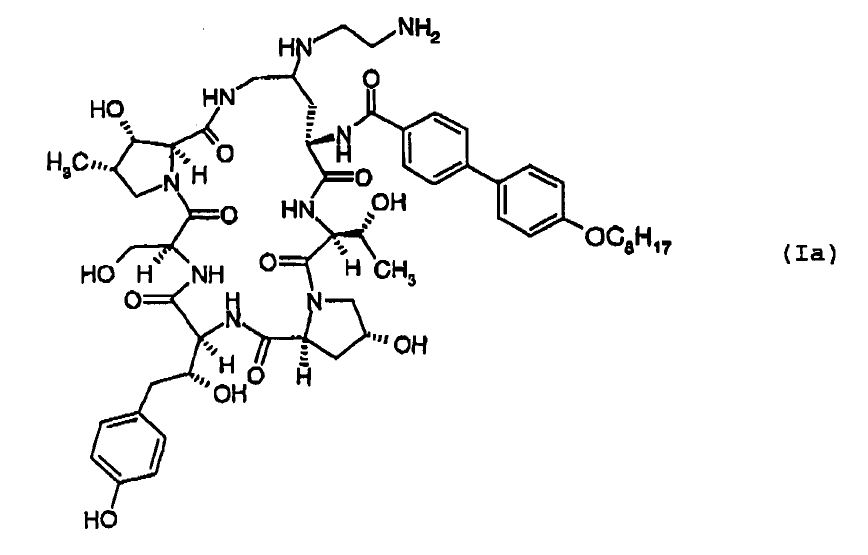 das die folgenden Schritte umfasst:
a) Eine Verbindung der Formel (II) 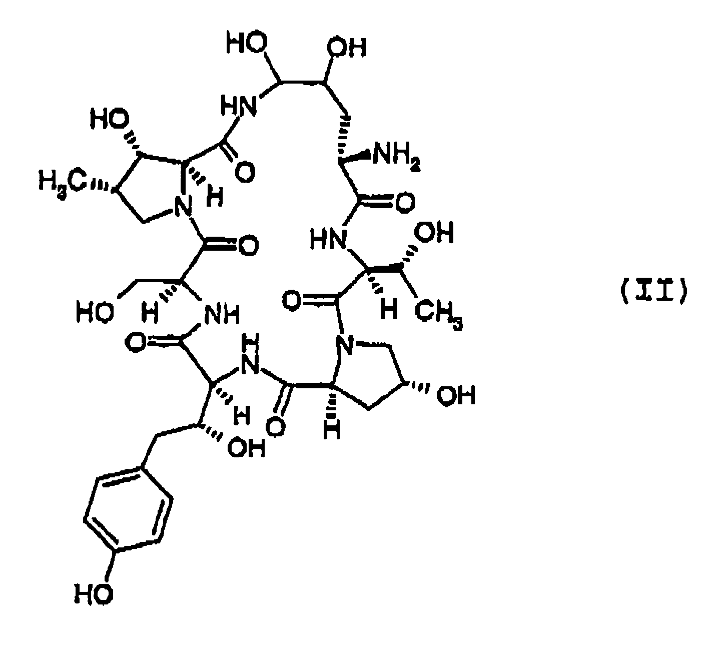 wird der Wirkung einer Säure der Formel C₈H₁₇-O-Ph-Ph-CO₂H unterzogen, wobei die Säure gegebenenfalls in aktivierter, isolierter oder nicht isolierter Form vorliegt, um die Verbindung der folgenden Formel (IIIa) zu erhalten: 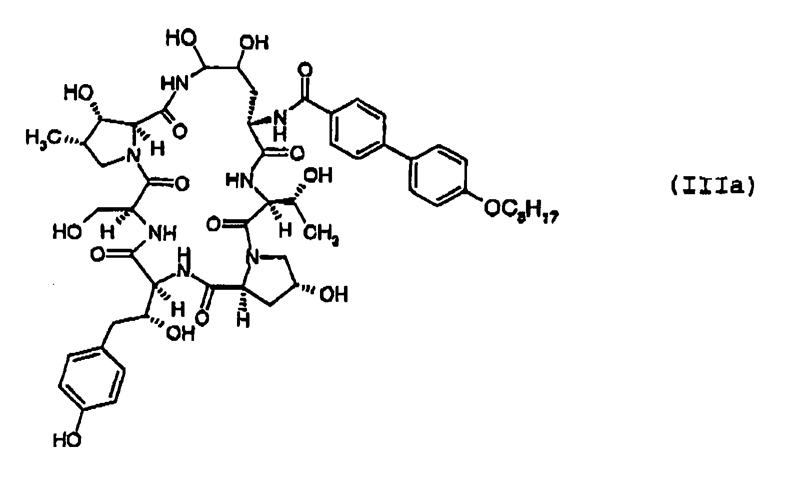
b) Die Verbindung der Formel (IIIa) wird gegebenenfalls einer Alkylierungsreaktion des Alkohols an Position 5 durch die Wirkung von Methanol in Gegenwart von PPTS unterzogen, um die Verbindung der folgenden (III'a) zu erhalten: 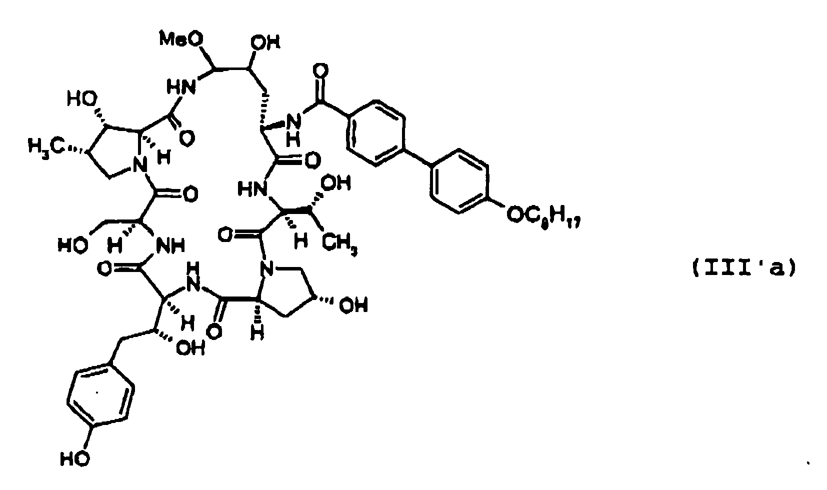
c) Die Verbindung der Formel (IIIa) oder (III'a) wird einer Dehydrierungsreaktion unterzogen, um ein Verbindung der folgenden Formel (IVa) zu erhalten: 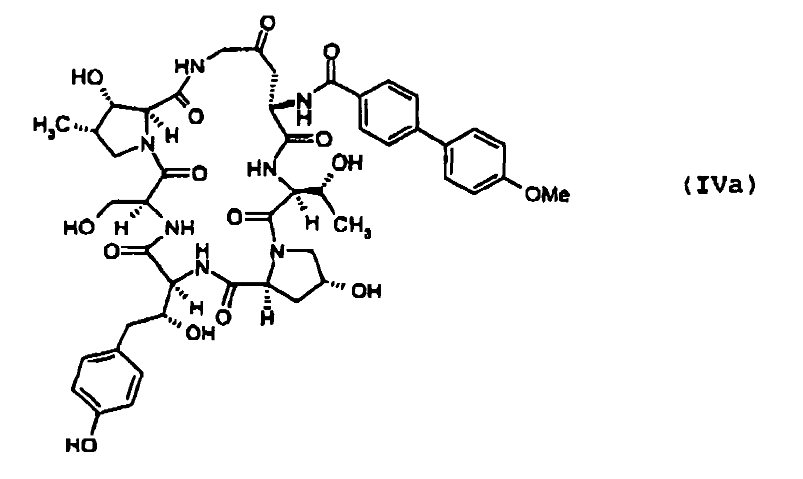
d) Die Verbindung der Formel (IVa) wird einer reduzierenden Aminierungsreaktion durch die Wirkung von Ethylendiamin in Gegenwart eines Reduktionsmittels, wie etwa NaBH₃CN, gekoppelt mit TiCl₄, NaH (Boc-L-pro)₃ oder NaH (Bzl-L-Pro)₃ unterzogen, um die Verbindung der Formel (Ia) zu erhalten, wie weiter oben definiert, die hauptsächlich eines der aktiven Isomere umfasst, wobei die Verbindung der Formel (Ia) einem oder mehreren der folgenden Arbeitsgänge unterzogen wird:
- Chromatographie
- Kristallisation
- Wirkung einer Base
- Salzbildung durch Wirkung von Chlorwasserstoffsäure.

5. Verfahren nach Anspruch 4, **dadurch gekennzeichnet, dass** die Verbindung der Formel (Ia) nacheinander den folgenden Arbeitsgängen unterzogen wird:
a) Reinigung durch Chromatographie auf Siliciumdioxid, dann Umkehrphasenchromatographie unter Verwendung eines Gemischs aus organischen Lösemitteln, Wasser und Trifluoressigsäure, um das Trifluoressigsäuresalz der Verbindung der Formel (Ia) zu erhalten
b) Wirkung einer Base, zum Beispiel durch Wirkung einer wässrigen Natriumbicarbonatlösung, um die Verbindung der Formel (Ia) in Form einer Base zu erhalten,
c) Salzbildung durch Wirkung von Chlorwasserstoffsäure, um das entsprechende Salz, nämlich das Dihydrochlorid der Verbindung der Formel (Ia) zu erhalten.

6. Verfahren nach einem der Ansprüche 1 bis 5, **dadurch gekennzeichnet, dass** die Aktivierungsreaktion der Säure in Gegenwart von Pentafluorphenol, N-Hydroxysuccinimid oder optional N-Hydroxybenzotriazol durchgeführt wird.

7. Verfahren nach einem der Ansprüche 1 bis 5, **dadurch gekennzeichnet, dass** die Acylierungsreaktion in Gegenwart einer isolierten aktivierten Säure in Gegenwart von Diisopropylethylamin durchgeführt wird.

8. Verfahren nach einem der Ansprüche 1 bis 5, **dadurch gekennzeichnet, dass** die Aktivierungsreaktion, dann die Acylierungsreaktion in Gegenwart einer nicht isolierten aktivierten Säure in Gegenwart von N-Methylpyrrolidon und DMTMM durchgeführt wird.

9. Verfahren nach einem der Ansprüche 1 bis 5, **dadurch gekennzeichnet, dass** die Dehydrierungsreaktion in Gegenwart von BAIB und gegebenenfalls von MgI₂ durchgeführt wird.

10. Verfahren nach einem der Ansprüche 1 bis 7, **dadurch gekennzeichnet, dass** die Dehydrierungsreaktion in Gegenwart von HBr-AcOH und von MgI₂ durchgeführt wird.

11. Verfahren nach den Ansprüchen 9 und 10, **dadurch gekennzeichnet, dass** das Produkt, das aus der Dehydrierung stammt, durch Kristallisation in einem DMF/Aceton- oder DMF/AcOEt-Gemisch gereinigt wird.

12. Verfahren nach einem der Ansprüche 1 bis 5, **dadurch gekennzeichnet, dass** die reduzierende Aminierungsreaktion in Gegenwart eines Reduktionsmittels durchgeführt wird, das aus NaBH₃CN, gekoppelt mit TiCl₄, NaH(Boc-L-pro)₃ und NaBH(Bzl-L-Pro)₃ ausgewählt ist.

13. Verfahren zur Herstellung einer Verbindung der Formel (IV) oder (IVa) durch eine Dehydrierungsreaktion der Verbindungen der Formeln (III') oder (III'a), wie in einem der Ansprüche 1 bis 4 und 9 bis 11 definiert.

14. Verfahren zur Herstellung einer Verbindung der Formel (I) oder (Ia) durch eine reduzierende Aminierungsreaktion der Verbindungen der Formeln (IV) oder (IVa), wie in einem der Ansprüche 1 bis 4 und 12 definiert.

15. Als Intermediärverbindungen der Verbindungen der Formeln (III') oder (III'a), wie in den Ansprüchen 1 und 4 definiert.

16. Als Intermediärverbindungen der Verbindungen der Formeln (IV'), wobei R so ist, wie in einem der Ansprüche 1 bis 2 definiert, und (IV'a), wenn R für Ph-Ph-OC₈H₁₇ steht:
